(19) Europäisches Patentamt / European Patent Office / Office européen des brevets

(11) **EP 3 682 033 B1**

(12) **EUROPEAN PATENT SPECIFICATION**

(45) Date of publication and mention
of the grant of the patent:
**03.12.2025 Bulletin 2025/49**

(21) Application number: **18773561.8**

(22) Date of filing: **13.09.2018**

(51) International Patent Classification (IPC):
**C12Q 1/6874** (2018.01)

(52) Cooperative Patent Classification (CPC):
(C-Sets available)
**C12Q 1/6874** (Cont.)

(86) International application number:
**PCT/GB2018/052598**

(87) International publication number:
**WO 2019/053430 (21.03.2019 Gazette 2019/12)**

(54) **SEQUENCING OF NUCLEIC ACID MOLECULES**

SEQUENZIERUNG VON NUKLEINSÄUREMOLEKÜLEN

SÉQUENÇAGE DE MOLÉCULES D'ACIDE NUCLÉIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.09.2017 GB 201714748**

(43) Date of publication of application:
**22.07.2020 Bulletin 2020/30**

(73) Proprietor: **Oxford University Innovation Ltd.
Oxford, Oxfordshire OX2 0JB (GB)**

(72) Inventors:
• **KAPANIDIS, Achillefs
Oxford
Oxfordshire OX2 0JB (GB)**
• **ANDREWS, Rebecca
Oxford
Oxfordshire OX2 0JB (GB)**

(74) Representative: **Barker Brettell LLP
100 Hagley Road
Edgbaston
Birmingham, West Midlands B16 8QQ (GB)**

(56) References cited:
WO-A1-00/04193        WO-A1-00/04193
WO-A2-97/19193        US-A- 6 090 549
US-B1- 6 440 671

• **ANDREW TATON T ET AL: "Scanometric DNA
Array Detection with Nanoparticle Probes",
SCIENCE, AMERICAN ASSOCIATION FOR THE
ADVANCEMENT OF SCIENCE, US, vol. 289, 8
August 2000 (2000-08-08), pages 1757 - 1760,
XP002158394, ISSN: 0036-8075, DOI: 10.1126/
SCIENCE.289.5485.1757**
• **ANDREW TATON T ET AL: "Scanometric DNA
Array Detection with Nanoparticle Probes",
SCIENCE, AMERICAN ASSOCIATION FOR THE
ADVANCEMENT OF SCIENCE, US, vol. 289, 8
August 2000 (2000-08-08), pages 1757 - 1760,
XP002158394, ISSN: 0036-8075, DOI: 10.1126/
SCIENCE.289.5485.1757**

Remarks:
The complete document including Reference
Table(s) and the Sequence Listing(s) can be
downloaded from the EPO website

EP 3 682 033 B1

(52) Cooperative Patent Classification (CPC): (Cont.)

C-Sets
**C12Q 1/6874, C12Q 2525/101, C12Q 2549/113,
C12Q 2563/107, C12Q 2565/501**

**Description**

The present invention relates to a method of sequencing of a nucleic acid molecule and a method of single-molecule phenotyping and sequencing of a nucleic acid molecule.

Single-molecule DNA sequencing methods have made important contributions to genomics, diagnostics and functional analysis of genetic processes. Despite the fact that there are a large variety of single-molecule sequencing methods, there is currently no proficient, high-throughput way to connect the functional properties of a single DNA molecule (single-molecule phenotype) with DNA sequence.

Within the last decade, single-molecule sequencing has become established in the commercial long-read sequencing market due to two companies, Pacific Biosciences and Oxford Nanopore Technologies, both with differing approaches. Pacific Biosciences use a single immobilised polymerase within a zero-mode waveguide to incorporate fluorescently labelled nucleotides (nts) during DNA synthesis allowing single bases to be read in real-time. Whereas, Oxford Nanopore Technologies' DNA sequencing method utilises the potential difference change across a protein pore as nucleotides pass through. Both major single-molecule sequencing methods require specialist equipment and cannot directly link single-molecule phenotype with DNA sequence.

The scientific publication Taton et al., 'Scanometric DNA Array Detection with Nanoparticle Probes', Science (2000), Vol 289, Issue 5485, pp. 1757-1760, DOI: 10.1126/science.289.5485.1757, discloses a method for analysing combinatorial DNA arrays using oligonucleotide-modified gold nanoparticle probes.

US patent US6440671B1 discloses methods for detecting disease-associated alleles in patient genetic material, whereby nucleic acid duplexes are formed. Methods for determining the length of a repeat sequence in DNA/RNA, and for determining the base sequence of unknown DNA/RNA, are also disclosed therein.

International patent application WO0004193A1 discloses compositions and methods for detecting single nucleic acid molecules using rolling circle amplification (RCA) of single-stranded circular templates primed by immobilized primers.

International patent application WO9719193A2 discloses compositions and methods for amplification of and multiplex detection of molecules of interest involving rolling circle replication, employed to simultaneously detect multiple specific nucleic acids in a sample.

There is need to overcome at least the above problems and to provide alternative, and preferably improved, methods for sequencing single nucleic acid molecules and molecule phenotyping and sequencing of a nucleic acid molecule.

According to a first aspect of the present invention, there is provided a method of sequencing a nucleic acid molecule, in accordance with claim 1 herein.

Also described is a method of sequencing a nucleic acid molecule, the method comprising:

(a) providing the nucleic acid immobilised on a surface;

(b) forming a single-stranded gap section by partially duplexing the nucleic acid such that the single-stranded gap section is flanked by duplex sections, wherein the sequence to be sequenced is a sequence of the single-stranded gap section;

(c) providing a set of at least four fluorescently labelled oligonucleotide probes, and each probe comprising:

(i) a nucleotide X at a position arranged to oppose a nucleotide of the gap section of the immobilised nucleic acid, wherein such nucleotide is the interrogated nucleotide, and

(ii) universal or degenerate bases arranged to oppose some or all of the remaining nucleotide positions of the gap section,

wherein the at least four fluorescently labelled oligonucleotide probes comprise:

- a first fluorescently labelled oligonucleotide probe wherein nucleotide X is a cytosine;
- a second fluorescently labelled oligonucleotide probe wherein nucleotide X is a guanine:
- a third fluorescently labelled oligonucleotide probe wherein nucleotide X is an adenine; and
- a fourth fluorescently labelled oligonucleotide probe wherein nucleotide X is a thymine or uracil;

(d) detecting binding, or absence thereof, of the fluorescently labelled oligonucleotide probes with the gap section of the immobilised nucleic acid, wherein the identity of the interrogated nucleotide of the gap section of the immobilised nucleic acid is identified as the complementary base of the nucleotide X of the fluorescently labelled oligonucleotide probe that has the highest incidence of binding;

(e) repeating steps (c) and (d) for interrogating subsequent nucleotide positions of the gap section of the immobilised nucleic acid until sufficient nucleotides of the gap section have been identified to be able to determine a sequence.

The invention advantageously provides the ability to sequence a nucleic acid at a single-molecule level and in a form that can be linked to functional studies of the same nucleic acid molecule, or molecules linked thereto. In particular, due to the immobilisation of the nucleic acid on a surface, the observed functional/phenotypic affects associated with the same surface location can be attributed to the nucleic acid sequence. Using large libraries of nucleic acids (such as DNA and RNA oligonucleotides), this capability allows the identification of the sequence-dependence of processes that operate on DNA or RNA substrates, the discovery of DNA/RNA aptamers for desirable binding/reaction profiles versus various targets (such as proteins, nucleic acids, small molecules etc.), and the reading of bar-codes that can be introduced in biological molecules and cells.

The invention further advantageously allows the ability to introduce 4^N randomised sequences into nucleic acids, which are then fixed on a surface, and then studied for interactions with individual biomolecules such as proteins, and then sequenced. For example, a skilled person can create a library of DNA molecules with 4^N randomized sequence members (e.g. 4^5) using automated DNA synthesis and degenerate nucleotides for the section to be randomized. DNAs can then be converted into a double-stranded form and can be labelled or modified using suitably modified primers and PCR. The library can then be immobilised on a solid support such as a glass coverslip for functional interrogation at the single-molecule level (to obtain the single-molecule phenotype).

The randomised sequences can also be introduced in modified genes, that are introduced to cells, which are then fixed on a surface and then interrogated for functionality, and DNA sequence. For example, a skilled person can create a library with 4^N randomized sequence members (e.g. 4^5), which can then be integrated into a plasmid library. The randomised region is flanked from either side with two sequences that are long enough and unique relative to the genome of an organism of interest, allowing the generation of a single-stranded gap section for sequencing after phenotyping at a cellular level. Transformation of these fragments can lead to populations of cells (e.g., *E. coli*), with each cell making one of many permutations of a certain protein according to the introduced 4^N randomised sequences. The protein may be any protein that has a cellular phenotype, such as a protein that kills bacteria or changes their expression. Fixing the cells to a surface (thereby immobilising their nucleic acid content to a surface) followed by sequencing according to the present description allows the link to be established between a cell having a certain phenotype and the nucleic acid sequence associated with that phenotype. The fixed cell(s) may be permeabilised prior to the sequencing in order to allow sequencing reagent access to the immobilised nucleic acid. Vvedenskaya et al. (Molecular Cell 60, 953-965, 2017) is a publication that makes such libraries on plasmid, transforms them in bacteria, and then sequences the nucleic acid by a different method using next-generation sequencing. However, such a method does not provide phenotypic analysis and sequencing at the single-cell level, or the single-molecule level, by fixing the cells to a surface prior to sequencing their nucleic acid content.

**In** one embodiment, steps (c) and (d) are repeated until the gap section of the immobilised nucleic acid is fully sequenced.

Interrogating subsequent nucleotide positions of the gap section of the immobilised nucleic acid may comprise providing a further set of at least four fluorescently labelled oligonucleotide probes, wherein the position of nucleotide X is changed to oppose a different/subsequent nucleotide to be interrogated of the immobilised nucleic acid.

Detecting binding, or absence thereof, by the set of at least four fluorescently labelled oligonucleotide probes may be done sequentially one after the other (i.e. the detection of hybridisation of each probe may be done in the absence of other probes of the probe set), for example in embodiments wherein the probes comprise the same fluorescent label. In another embodiment, the set of at least four fluorescently labelled oligonucleotide probes may be provided together, or in sub-groups thereof, for example when a different fluorophore is used for the different probes such that they are distinguishable from each other. Therefore, in one embodiment, the at least four fluorescently labelled oligonucleotide probes each comprise a different fluorescent label such that they are distinguishable from each other.

Detecting the binding, or absence thereof, of the fluorescently labelled oligonucleotide probes with the gap section of the immobilised nucleic acid may comprise detecting the transient binding, or absence thereof, of the fluorescently labelled oligonucleotide probes with the gap section of the immobilised nucleic acid. Detecting the binding, or absence thereof, of the fluorescently labelled oligonucleotide probes with the gap section of the immobilised nucleic acid may comprise detecting the specific binding, or absence thereof, of the fluorescently labelled oligonucleotide probes with the gap section of the immobilised nucleic acid.

The identity of the interrogated nucleotide of the gap section of the immobilised nucleic acid may be identified as the complementary base of the nucleotide X of the fluorescently labelled oligonucleotide probe that has the highest level of binding, or highest level of transient binding as appropriate.

In one embodiment, the single-stranded gap section is flanked by a pair of duplex sections. In one embodiment, partially duplexing the nucleic acid comprises hybridising a pair of gap-flanking nucleic acid strands that are each complementary to a different section of the nucleic acid to be duplexed, wherein the single-stranded gap section is formed therebetween. In another embodiment, the partially duplexed nucleic acid comprising a single stranded gap-section is formed by circularising the nucleic acid with a splint nucleic acid that is arranged to hybridise to each end of the nucleic acid (i.e. forming a bridge between each end to form a circularised partially duplexed nucleic acid molecule comprising a single stranded gap-section).

In another embodiment, partially duplexing the nucleic acid comprises forming a hairpin loop in the nucleic acid, such that the nucleic acid partially hybridises with itself. A pair of hairpin loops may be formed at opposing ends of the nucleic acid, wherein the single-stranded gap section is formed therebetween. Alternatively, a hairpin loop may be formed at one end/section of the nucleic acid, and a gap-flanking nucleic acid strand that is complementary to a section of the nucleic acid to be duplexed may be hybridised to the nucleic acid, wherein the single-stranded gap section is formed between the duplex formed by the hairpin, and the duplex formed by the hybridised gap-flanking nucleic acid strand.

The gap-flanking nucleic acid strands may have a predetermined/known sequence. The sequences of the nucleic acid to be duplexed may determine the complementary sequences of the gap-flanking nucleic acid strand(s), or in an embodiment requiring a hairpin loop, determine the section of the nucleic acid to be looped back and hybridise to form the hairpin loop.

The sequences of the nucleic acid to be duplexed may be designed or built into the nucleic acid, such that they flank a sequence of unknown/random nucleotides to be interrogated. For example, the sequences of the nucleic acid to be duplexed may be designed or built into the nucleic acid during the synthesis of the nucleic acid. In another example, the sequences of the nucleic acid to be duplexed may be built into the nucleic acid during recombinant assembly of the nucleic acid.

The duplexing of the nucleic acid may be arranged to create the single-stranded gap section. The single-stranded gap section can be placed in the middle of the nucleic acid sequence, or closer to the ends of the nucleic acid. The single-stranded gap section can also be formed in circular nucleic acid molecules. The positioning of the single-stranded gap section (i.e. middle or near to an end) may depend on the nature of a functional assay that may be performed prior to the sequencing of the nucleic acid.

The sequencing may be done at, or around, room temperature (i.e. between 18°C and 28°C, such as about 24°C). The sequencing may be done at a temperature of between 5°C and 45°C. Different temperatures will allow the utilisation of gaps of different length.

Different buffer components can be provided to facilitate the sequencing. In one embodiment, a monovalent ion concentration ($Na^+$) can be provided at a concentration of between about 0.01 and about 3 M. Adjusting the monovalent ion concentration ($Na^+$) can change the binding affinity of the sequencing probes to the single-stranded gap section; high salt concentration screen electrostatic repulsions and therefore enhance the stability of hybridised nucleic acids. An additive TMAC can be used, for example at 3M concentrations, to make A-T rich sequences equivalent in stability to G-C rich sequences.

During phenotypic and sequencing assays, buffer components are chosen by the skilled person to enhance the observation of the binding or reaction in question. To allow extended observations, oxygen scavengers (such as glucose oxidase/catalase/glucose, or PCA/PCD) and triplet-state quenchers (such as TROLOX and mercaptoethylamine) can be used to extend the photobleaching lifetime of the fluorophores, and to minimise complicating photophysics. Therefore, in one embodiment, the sequencing reaction is provided with an oxygen scavenger and/or triplet-state quencher.

In one embodiment, the fluorescently labelled oligonucleotide probes comprise universal bases arranged to oppose some or all of the remaining nucleotide positions of the gap section. The skilled person will recognise that a universal base can be described as a base analog without hydrogen bonding groups that can pack efficiently in duplex DNA, but shows no or insignificant selectivity in pairing with native basses, such as A, C, G, and T. Examples of universal bases are inosine, deoxyinosine, 5' nitroindole, and 3-nitropyrrole. Several universal bases are also described by Berger et al. (Nucleic Acids Res. 2000 Aug 1; 28(15): 2911-2914). In one embodiment, the universal bases may comprise or consist of inosine.

In another embodiment, the fluorescently labelled oligonucleotide probes comprise degenerate bases arranged to oppose some or all of the remaining nucleotide positions of the gap section. The term "degenerate bases" used herein means any base, such as any of A, C, T, or G. The degenerate base may or may not match the opposing base of the immobilised nucleic acid in the gap section.

The fluorescently labelled oligonucleotide probes may be equal in length to the gap section of the immobilised nucleic acid. In another embodiment, the fluorescently labelled oligonucleotide probes may be shorter or longer in length to the gap section of the immobilised nucleic acid. The fluorescently labelled oligonucleotide probes may be at least one nucleotide in length (i.e. may consist of a single labelled nucleotide). The fluorescently labelled oligonucleotide probes may be at least 2 nucleotides in length. The fluorescently labelled oligonucleotide probes may be at least 3 nucleotides in length. The fluorescently labelled oligonucleotide probes may be at least 4 nucleotides in length. The fluorescently labelled oligonucleotide probes may be at least 5 nucleotides in length. The fluorescently labelled oligonucleotide probes may be at least 6 nucleotides in length. The fluorescently labelled oligonucleotide probes may be 1-12 nucleotides in length. The fluorescently labelled oligonucleotide probes may be 2-12 nucleotides in length. The fluorescently labelled oligonucleotide probes may be 3-12 nucleotides in length. The fluorescently labelled oligonucleotide probes may be 6-12 nucleotides in length. The fluorescently labelled oligonucleotide probes may be 1, 2, 3, 4, 5, 6, 7, 8, 9, 10, 11 or 12 nucleotides in length. Use of conditions that destabilise nucleic acid hybrids (e.g., low $Na^+$ concentration) will also allow longer gaps (e.g. up to 20nts) to be used. Therefore, in another embodiment, the fluorescently labelled oligonucleotide probes are 13, 14, 15, 16, 17, 18, 19, 20, or more nucleotides in length.

In one embodiment, the fluorescently labelled oligonucleotide probes comprise or consist of DNA. In another embodiment, the fluorescently labelled oligonucleotide probes comprise or consist of RNA. In another embodiment, the fluorescently labelled oligonucleotide probes comprise or consist of nucleic acid analogue such as PNA, LNA or PMO, or combinations thereof. The fluorescently labelled oligonucleotide probes may comprise or consist of nucleic acid selected from DNA, RNA, and nucleic acid analogues, or combinations thereof.

The fluorescently labelled oligonucleotide probes may comprise further structural or identification sequences (e.g. barcode sequence) that is not arranged to hybridise to, oppose, or complement the sequence of the gap section of the immobilised nucleic acid.

The fluorescent labels of the fluorescently labelled oligonucleotide probes may be selected from any of the group comprising 5-carboxyfluorescein (FAM), tetramethylrhodamine (TMR), Alexa-Fluor fluorophores (such as Alexa488, Alexa532, Alexa546, Alexa555, Alexa568, Alexa594, and Alexa647; available from Molecular Probes/Invitrogen), BODIPY dyes, ATTO dyes (such as ATTO532, ATTO568, ATTO594, ATTO647n, and ATTO655; available from Attotech) and cyanine dyes (such as Cy2, Cy3, Cy3B, Cy3.5, Cy5, Cy5.5, and Cy7; available from GE Healthcare), or combinations thereof.

In one embodiment, the binding, such as transient binding, of a fluorescently labeled oligonucleotide probe to the gap-section of the nucleic acid is detected by measurement of Förster resonance energy transfer (FRET) between the fluorophore on the fluorescently labeled oligonucleotide probe and a second fluorophore. The second fluorophore may be tagged on the immobilized nucleic acid molecule or duplex thereof. For example, the second fluorophore may be tagged on a gap-flanking nucleic acid strand that is hybridized to the immobilized nucleic acid molecule. For example, the fluorophores Cy3B and ATTO647N can be used together as a donor and an acceptor pair for FRET. Therefore, the fluorophore on the fluorescently labeled oligonucleotide probe may comprise Cy3B, and the second fluorophore may comprise ATTO647N, or vice versa.

A second fluorophore or any other label of the probes/seals can also be employed. In one embodiment, the 2nd fluorophore allows FRET where both fluorophores are on the probe (otherwise known as "seal" herein) and the quenching state is dependent on being duplexed and far apart (thus resulting in low FRET efficiency), or non-duplexed and fluorophores brought together (thus resulting in contact-mediated quenching). The FRET acceptor can either be a high-fluorescent fluorophore (such as ATTO647N); in this case, signals in both FRET donor and FRET acceptor emission channels will be used to monitor binding. The FRET acceptor can also be a dark (non-fluorescent) quencher (such as QSY7, QSY21, BHQ2 or BHQ3); in this case, the signals in the FRET donor emission channels will be used to monitor binding. Where there are two fluorophores tagged on an oligonucleotide probe, the fluorophores may be at opposite ends of the probe, or at least sufficiently far apart that the length of nucleic acid therebetween eliminates contact-mediated quenching upon binding, while allows efficient FRET between donor and acceptor upon binding of the distance between the fluorophores may be at least 5 or more nucleotides. The skilled person can provide an appropriate distance depending on the exact pair of fluorophores used (their structure, dynamics, and spectral properties).

The fluorescently labelled oligonucleotide probes may be provided at a concentration of between about 0.1 nM and about 10 $\mu$M. The fluorescently labelled oligonucleotide probes may be provided at a concentration of about 10 nM. In another embodiment, the fluorescently labelled oligonucleotide probes may also be provided at a concentration of about 500 nM. In another embodiment, the fluorescently labelled oligonucleotide probes may be provided at a concentration of between about 5 nM and about 1000nM. In another embodiment, the fluorescently labelled oligonucleotide probes may be provided at a concentration of between about 10 nM and about 800nM. In another embodiment, the fluorescently labelled oligonucleotide probes may be provided at a concentration of between about 100 nM and about 800nM. In another embodiment, the fluorescently labelled oligonucleotide probes may be provided at a concentration of between about 300 nM and about 800 nM. In another embodiment, the fluorescently labelled oligonucleotide probes may be provided at a concentration of between about 400 nM and about 600 nM. In another embodiment, the fluorescently labelled oligonucleotide probes may be provided at a concentration of between about 5 nM and about 50nM. In another embodiment, the fluorescently labelled oligonucleotide probes may be provided at a concentration of between about 5 nM and about 20nM. In another embodiment, the fluorescently labelled oligonucleotide probes may be provided at a concentration of between about 8 nM and about 15nM. In another embodiment, the fluorescently labelled oligonucleotide probes may be provided at a concentration of between about 10 nM and about 20nM. The concentrations or relative amounts of the at least four fluorescently labelled oligonucleotide probes may be substantially equal to each other. In another embodiment, the fluorescently labelled oligonucleotide probes may be provided at a concentration of greater than 100nM, or greater than 200nM. The provided concentrations of fluorescently labelled oligonucleotide probes refers to the concentration of total probe species unless stated otherwise i.e. not the concentration of a singly fluorescently labelled oligonucleotide probe.

The concentration of fluorescently labelled oligonucleotide probes determines the rate of binding of the probes to the immobilised DNA. Higher concentrations (e.g., >100 nM) enhance the rate of binding, but contribute to high fluorescence background, and prevent or make difficult the detection of single immobilised molecules. Lower concentrations are associated with low background, but lead to increased waiting times for collecting sufficient binding events for base calling. Other techniques minimised this background either by suppressing background fluorescence via confined function/detection (such as with zero-mode waveguides) or by adopting label-free approaches (such as nanopores). The present invention advantageously can be used with a high concentration range that allows rapid detection without the need for specialised structures such as zero-mode waveguides.

The skilled person will understand that the concentration of the probe(s) and/or the assay time may be adjusted depending on the nature of the probe (i.e., the degree of base pairing, the G/C content, the length of the single stranded gap-section, and the degree of quenching/dequenching achieved upon binding of the probe to its target). The concentration and/or assay time adjustment may also depend on the use of universal or degenerate bases, since in the case of universal bases, a large fraction of the fluorescently labelled oligonucleotide probes (25%) will be able to bind preferentially to the interrogated sequence, whereas in the case of degenerate bases, a much smaller fraction of the fluorescently labelled oligonucleotide probes (1 out of $4^N$ bases) will be able to bind preferentially to the interrogated sequence. Therefore, in an embodiment wherein universal bases are provided in the fluorescently labelled oligonucleotide probes, the concentration of the fluorescently labelled oligonucleotide probes may be between 10 nM and 500 nM (total probe concentration). **In** an embodiment wherein degenerate bases are provided in the fluorescently labelled oligonucleotide probes, the concentration of the fluorescently labelled oligonucleotide probes may be between 10 nM and 500 nM (total probe concentration), and the assay may be performed for a longer time to account for the less frequent binding of the complementary seal DNAs to the immobilised target.

**In** one embodiment, the nucleic acid is synthetically produced. Additionally the nucleic acid may be non-natural/synthetic in origin (i.e. comprises non-wild-type sequence). **In** another embodiment, the nucleic acid may be recombinantly produced *in vivo,* for example in a cell. In another embodiment, the nucleic acid may be naturally occurring *in vivo,* for example in a cell.

In one embodiment, the nucleic acid comprises a combination of pre-determined sequence and unknown/randomised sequence. The nucleic acid may comprise a pair of pre-determined sequences flanking an unknown/randomised sequence therebetween. The predetermined sequences may be arranged to form a duplex with gap-flanking nucleic acid strands.

The nucleic acid may be provided in the form of a library of nucleic acids comprising a randomised nucleotide region. In one embodiment, the library has $4^N$ members, where $N$ is the number of randomised bases, for example, a randomised 5-nucleotide region will be represented by 1024 possible DNA sequences and a randomised library of 9-nucleotide region will be represented by $2.6 \times 10^5$ different sequences. In one embodiment, $N$ is 5. In another embodiment, $N$ is 9. In another embodiment, $N$ is 1, 2, 3, 4, 5, 6, 7, 8, 9, 10, 11, or 12. In addition to the randomised nucleotide region, the library members may be provided with known/pre-determined sequence. The randomised nucleotide region may be flanked by known/pre-determined sequences. The known/pre-determined sequences may be identical for each library member. The known/pre-determined sequences may comprise the sequences that are arranged to form a duplex with the gap-flanking nucleic acid strands. The library members may be labelled with a fluorescent oligonucleotide probe, preferably the same fluorescent oligonucleotide probe for each member.

Advantageously, the use of small (e.g. 9 nts) gap-sections can require libraries of short (e.g. 9 nts) fluorescently labelled oligonucleotide probes (otherwise known as "seals"); the short size can prevent stable hybridization between unbound short fluorescently labelled oligonucleotide probes in solution. The formation of the gap also removes secondary structures that may be present on the template, addressing a significant issue with templates such as RNA. The gap also allows simple DNA (i.e., not only DNA nanostructures) to be interrogated for binding.

The transient binding can also work with short single-stranded DNAs that can be prepared after the functional assay via restriction digestion and removal of the non-surface-immobilised strand of the short dsDNA fragment. The assay can also work with template-primer hybrids that leave a short overhang (with a size similar to the preferred gap size, i.e., 6-12 nts) on one of the ends.

In one embodiment, the nucleic acid comprises or consists of DNA. In another embodiment, the nucleic acid comprises or consists of RNA. In another embodiment, the nucleic acid comprises or consists of nucleic acid analogue such as PNA, LNA or PMO, or combinations thereof. The nucleic acid may be selected from DNA, RNA, and nucleic acid analogues, or combinations thereof.

The nucleic acid (for example each of the library members) may comprise a functionality (such as 5' or 3'-biotinylation of the DNA) that allows its surface-immobilisation on a surface. For example, the functionality may comprise a 5' or 3'-biotinylation of the nucleic acid to allow surface immobilisation on neutravidin-coated glass surfaces. In one embodiment, the nucleic acid is biotinylated. The skilled person will recognise that there are other methods to immobilise the nucleic acid, such as crosslinking it to surfaces decorated with reactive groups (e.g. $NH_2$-modified DNA covalently linked to NHS-modified surfaces; SH-modified DNA covalently linked to maleimide-modified surfaces; and azido-modified DNA covalently linked to alkyne-modified surface via clink chemistry, any of which may be used in the method according to the description), or using antibody/antigen interactions (e.g. digoxygenin-modified DNA covalently linked to anti-dig antibody-coated surfaces).

The biotin, or other functional group for immobilisation, may be provided on a linker. The linker may be sufficiently long enough to avoid steric hindrance. Polyethylene glycol (PEG) linkers may be used to provide a hydrophilic layer that can prevent/minimise non-specific adsorption to surfaces. PEG spacers of different sizes (e.g. PEG333, PEG5000) and different functionalities (e.g. lipid-PEG, poly-lysine PEG) can be used to further minimise non-specific binding.

The nucleic acid may be immobilised on a solid surface. The surface may comprise or consist of glass. The surface may be a plate, such as a flat plate. In one embodiment, the surface is a glass coverslip. The surface may be treated to facilitate immobilisation of the nucleic acid. In one embodiment, the surface, such as a glass surface, is neutravidin-coated.

In one embodiment, immobilised nucleic acids are spatially arranged onto the surface, such as glass. For example, single nucleic acid molecules or groups of the same species of nucleic acid molecules may be spaced apart from other nucleic acid molecules or groups. The spacing may be sufficient that they can be spatially resolved from each other, for example when detecting a fluorescent oligonucleotide probe emission. The fluorescent oligonucleotide probe emission may be detected and visualised by a single-molecule fluorescence microscope. Crowded-field analysis of immobilised fluorescent molecules may be used to detect and/or visualise the fluorescent oligonucleotide probe emissions. High-precision localisation of the binding events may be detected via identification of the centre of the point-spread function of the bound fluorophores.

The spatial arrangement may be random or patterned, for example into an array. The nucleic acid molecules may be sparsely immobilised on the surface to allow their detection on the surface at the single-molecule level, for example using a single-molecule fluorescence microscope.

In one embodiment, the nucleic acid molecules may be immobilised on the surface at a surface density of about 1 molecule per $\mu m^2$. In another embodiment the nucleic acid molecules may be immobilised on the surface at a surface density of about 10 molecules per $\mu m^2$, for example when crowded-field analysis of immobilised fluorescent molecules is used. In another embodiment, the nucleic acid molecules may be immobilised on the surface at a surface density of about 1000 molecules per $\mu m^2$, for example when employing high-precision localisation of the binding events (via identification of the centre of the point-spread function of the bound fluorophores). In another embodiment the nucleic acid molecules may be immobilised on the surface at a surface density of between about 1 and about 1500 molecules per $\mu m^2$. In another embodiment the nucleic acid molecules may be immobilised on the surface at a surface density of between about 1 and about 1100 molecules per $\mu m^2$. In another embodiment the nucleic acid molecules may be immobilised on the surface at a surface density of between about 1 and about 1000 molecules per $\mu m^2$. In another embodiment the nucleic acid molecules may be immobilised on the surface at a surface density of between about 1 and about 500 molecules per $\mu m^2$. In another embodiment the nucleic acid molecules may be immobilised on the surface at a surface density of between about 1 and about 100 molecules per $\mu m^2$. In another embodiment the nucleic acid molecules may be immobilised on the surface at a surface density of between about 1 and about 50 molecules per $\mu m^2$.

The use of a regular array for the deposition of DNA will increase the throughput and decrease the computational costs of sequencing, since it will provide spacing that is optimised for the microscope, fluorophores and binding times involved.

Advantageously, imaging on a single-molecule fluorescence microscope with a large field-of-view, such as with sCMOS-based wide-field imaging, allows parallel detection of 500-2000 single molecules per field-of-view. Instrumentation with lower magnification on a sCMOS camera allows 15,000 molecules to be visualised per field of view. Implementation on sCMOS-based wide-field imaging combined with high-precision localisation of the binding events allows ~$1.5 \times 10^7$ molecules to be visualised per field of view, providing extensive sequence coverage without the need for scanning, and opening a large number of potential applications.

In one embodiment, the immobilised nucleic acid may be immobilised to the bottom of a well, such as an array of zero-mode waveguides (for example, comprising sub-wavelength apertures of about 10-100 nm in diameter, about 100 nm in depth). This allows a higher concentration of probe to be used, providing faster binding to the gap DNA and faster readout of sequences.

In one embodiment, the immobilised nucleic acid may be immobilised by way of being *in situ* in a cell and the cell is chemically fixed (e.g., using 1-4% paraformaldehyde in phosphate-buffer saline), permeabilised (e.g., using treatment with ice-cold 90% methanol) to allow access of the sequencing reagents to the nucleic acid of the cell, and then attached to the surface (e.g., to a polylysine-coated surface or to an agarose pad). Alternatively, the cells can be immobilised by capture in microfluidic channels.

In another embodiment the nucleic acid or groups of identical nucleic acid molecules may be immobilised on a bead or a nanoparticle (such as a quantum dot or a fluorescent nanodiamond). The nucleic acid may be immobilised on a glass bead. For sequencing according to the description, the bead may be fixed into position on a surface. Beads comprising immobilised nucleic acid on their surface may be spatially arranged, such that beads are spaced apart. The spacing may be sufficient to resolve the beads for example when detecting a fluorescent probe emission.

The method of the description may further provide the step of amplifying the immobilised nucleic acid. The amplification may comprise a rolling circle amplification (RCA) of the immobilised nucleic acid. The surface-immobilised nucleic acid may be circularised using a splint nucleic acid molecule (for example a DNA molecule), to bring together and close the ends, followed by ligation. The splint nucleic may also be used as a primer for the RCA.

Amplification of the immobilised nucleic acid can increase the speed of the assay and decrease the concentration of fluorescent seal DNAs needed is to amplify the gap-section. RCA can produce >100 copies of the randomised sequences within an hour (Mohsen and Coll, Acc Chem Res, 2016); removal of the DNA polymerase and annealing of a single gap-flanking oligo (which essentially combines the two gap-flanking DNAs) provides >100 amplified gap-sections, but still maintaining the link between any initial phenotype analysis and the sequence. The abundance of targets (>100) will decrease the time needed for binding or the concentration of fluorescence seals needed for binding by a similar fold (~100-fold).

According to a second aspect of the present invention, there is provided a method of single-molecule phenotyping and sequencing of a nucleic acid molecule, in accordance with claim 8 herein.

According to another description, there is provided a method of single-molecule phenotyping and sequencing of a nucleic acid molecule, the method comprising:

a) providing the nucleic acid molecule immobilised to a surface;
b) testing for a phenotype/function of the immobilised nucleic acid molecule; and
c) determining the sequence of the immobilised nucleic acid molecule such that an observed phenotype/function is linked to the sequence, wherein the nucleic acid is sequenced by the method of sequencing according to the description herein.

In one embodiment, testing for a phenotype/function of the immobilised nucleic acid molecule may comprise testing for binding of the nucleic acid to a target molecule. The target molecule may comprise a small molecule, an oligopeptide, a polypeptide (such as an enzyme or a protein), a nucleic acid, or a lipid molecule.

The target molecule may be labelled for detection, such as fluorescently labelled. The fluorescent label of target molecule may be selected from any of the group comprising 5-carboxyfluorescein (FAM), tetramethylrhodamine (TMR), Alexa-Fluor fluorophores (such as Alexa488, Alexa532, Alexa546, Alexa555, Alexa568, Alexa594, and Alexa647; available from Molecular Probes/Invitrogen), BODIPY dyes, ATTO dyes (such as ATTO532, ATTO568, ATTO594, ATTO647n, and ATTO655; available from Attotech) and cyanine dyes (such as Cy2, Cy3, Cy3B, Cy3.5, Cy5, Cy5.5, and Cy7; available from GE Healthcare), or combinations thereof.

In one embodiment, the binding, such as transient binding, of a fluorescently labeled target molecule to the immobilised nucleic acid is detected by measurement of Förster resonance energy transfer (FRET) between the fluorophore on the fluorescently labeled target molecule and a second fluorophore. The second fluorophore may be tagged on the immobilized nucleic acid molecule or duplex thereof. For example, the second fluorophore may be tagged on a gap-flanking nucleic acid strand that is hybridized to the immobilized nucleic acid molecule. For example, the fluorophores Cy3B and ATTO647N can be used together as a donor and an acceptor pair for FRET. Therefore, the fluorophore on the fluorescently labeled target molecule may comprise Cy3B, and the second fluorophore may comprise ATTO647N, or vice versa.

The method of the description can be particularly useful for discovering DNA/RNA aptamers, and for investigating desirable binding/reaction profiles versus various targets (such as proteins, nucleic acids, small molecules etc.).

In one embodiment, the phenotyping/functional assay may comprise the determination of the interaction of the immobilised nucleic acid with a sequence-dependent DNA/RNA binding protein. The sequence-dependent DNA/RNA binding protein may comprise a transcription factor or a viral protein. The sequence-dependent processing of the nucleic acid by a nucleic-acid polymerase may be determined, such as by RNA polymerase or DNA polymerase. The interaction of the nucleic acid with another nucleic acid may be determined, e.g., another DNA or RNA strand. In an embodiment wherein the immobilised nucleic acid is an aptamer, the binding to a target protein, biomolecule or particle may be determined.

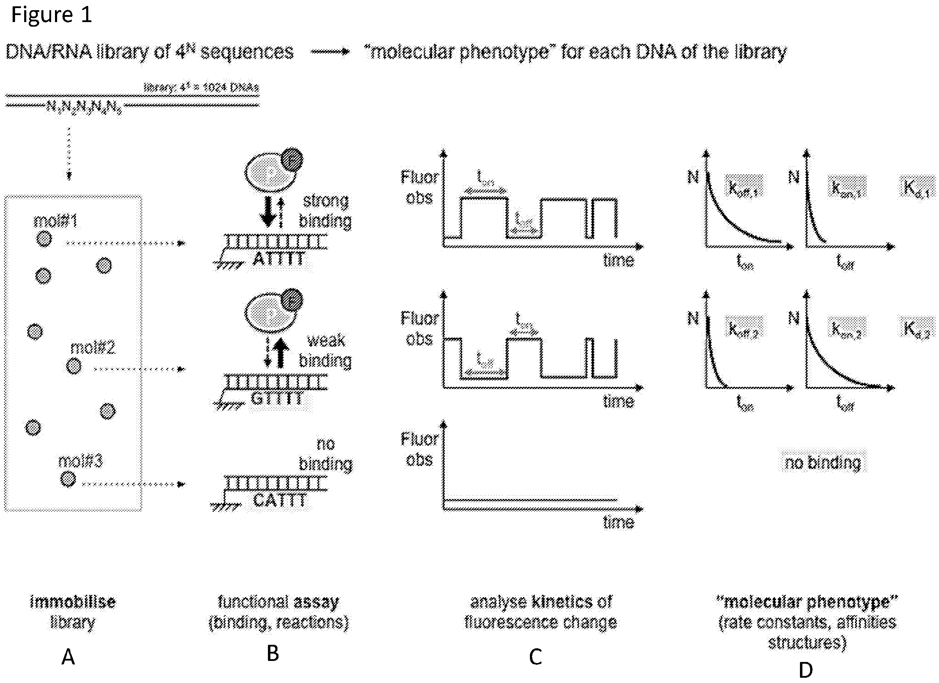

An example of functional assay that can be carried out according to the description is the binding and subsequent dissociation of a DNA-binding protein. The protein may be fluorescent; in such a case, binding of the protein to the DNA results (see Fig. 1C, top and middle) in a clear observable fluorescence, which is repeated binding events of the protein at the same location of the surface (see Fig. 1D).

The functional/phenotypical analysis may be on the double-stranded form of the nucleic acid, i.e. the fully double-stranded form not the partially duplexed form, which may be provided for sequencing. In one embodiment, the nucleic acid may be provided double-stranded. In one embodiment, the nucleic acid to be interrogated for function/phenotype is the gap section of the nucleic acid to be sequenced.

Providing the immobilised nucleic acid may comprise providing a library of nucleic acids, such as DNA, which is prepared using automated DNA synthesis with degenerate nucleotides inserted at the gap section of the nucleic acid to be interrogated for function/phenotype and to be sequenced. Providing the immobilised nucleic acid may comprise the same provision as for the method of sequencing according to the description herein.

Following the phenotypic analysis, the method may further comprise a denaturing and/or wash step, for example to remove any interacting or non-interacting molecules, prior to sequencing the nucleic acid. The wash step may comprise any suitable method for weakening binding between a nucleic acid and a bound molecule. In one embodiment, the wash step comprises washing the immobilised nucleic acid and its interacting partner in a high salt buffer.

The nucleic acid may be denatured to remove the top strand (i.e., the strand that is not directly immobilised/linked to the surface). Denaturing may be done chemically (e.g. using treatment with high NaOH concentration), thermally (e.g. by melting the strands by increasing temperature), or biochemically (e.g. using specialised nucleases).

An advantageous application of the method of the description is the ability to directly read the sequence of protein-DNA/RNA (ribonucleic acid) interactions and reactions. There are many different types of sequence dependent protein-DNA/RNA interactions. Some proteins interact with DNA/RNA by simply binding such as catabolite activator protein (CAP) and carbon storage regulator A protein (CsrA). Other proteins bind to the DNA and instigate reactions such as RNA polymerase initiating transcription. Other proteins bind to DNA as complex with an RNA and instigate reactions such as CRISPR-Cas9 initiating DNA cleavage and degradation. DNA/RNA also can be used to bind proteins, for example, aptamers (three-dimensional DNA/RNA), which bind and capture specific proteins, with many biomedical applications. There is a vast array of protein-DNA/RNA sequence dependent interactions that can be investigated.

According to a third aspect of the present invention, there is provided a method of single-molecule phenotyping and identification of a molecule, in accordance with claim 10 herein.

According to another description, there is provided a method of molecule phenotyping and identification of a molecule, wherein the molecule is tagged with a nucleic acid and identified by sequencing of the tagged nucleic acid, the method comprising:

a) providing the molecule immobilised to a surface;
b) testing for a phenotype/function of the immobilised molecule; and
c) determining the sequence of the tagged nucleic acid molecule such that an observed phenotype/function is linked to the tagged nucleic acid sequence, wherein the tagged nucleic acid is sequenced by the method of sequencing according to the description herein.

The immobilised molecule may comprise a protein that is fused (i.e. physically linked) to a nucleic acid. The nucleic acid may comprise nucleic acid that encodes the protein, such as its encoding mRNA. Such tagging of the nucleic acid is described in Roberts et al Current Opinion in Chem Bio 1999. Tagging the encoding mRNA of the protein may be performed using an *in vitro* translation system where the mRNA comprises an oligo linker of about 19-30 nt and a puromycin molecule at its 3'end. Upon reaching the oligo linker, the puromycin forms a covalent bond between the mRNA and its corresponding protein. Such tagging allows a direct link between measuring protein function in large libraries, and determination of the sequences associated with the desire function.

Testing for a phenotype/function of the immobilised molecule may comprise detection of an interaction, such as binding between the immobilised molecule and a target molecule.

The immobilised molecule may comprise a small molecule, an oligopeptide, a polypeptide (such as an enzyme or a protein), a nucleic acid, or a lipid molecule. The target molecule may comprise a small molecule, an oligopeptide, a polypeptide (such as an enzyme or a protein), a nucleic acid, or a lipid molecule.

The immobilised molecule and/or target molecule may be labelled for detection, such as fluorescently labelled. The fluorescent label of immobilised molecule and/or target molecule may be selected from any of the group comprising 5-carboxyfluorescein (FAM), tetramethylrhodamine (TMR), Alexa-Fluor fluorophores (such as Alexa488, Alexa532, Alexa546, Alexa555, Alexa568, Alexa594, and Alexa647; available from Molecular Probes/Invitrogen), BODIPY dyes, ATTO dyes (such as ATTO532, ATTO568, ATTO594, ATTO647n, and ATTO655; available from Attotech) and cyanine dyes (such as Cy2, Cy3, Cy3B, Cy3.5, Cy5, Cy5.5, and Cy7; available from GE Healthcare), or combinations thereof.

In one embodiment, the binding, such as transient binding, of a fluorescently labeled target molecule to the immobilised molecule is detected by measurement of Förster resonance energy transfer (FRET) between the fluorophore on the fluorescently labeled target molecule and a second fluorophore. The second fluorophore may be tagged on the immobilized molecule. For example, the fluorophores Cy3B and ATTO647N can be used together as a donor and an acceptor pair for FRET. Therefore, the fluorophore on the fluorescently labeled immobilised molecule may comprise Cy3B, and the second fluorophore on the target molecule may comprise ATTO647N, or vice versa.

According to a fourth aspect of the present invention, there is provided a method of single-cell phenotyping and sequencing of a nucleic acid molecule associated with the phenotype of a cell, in accordance with claim 11 herein.

According to another description, there is provided a method of single-cell phenotyping and sequencing of a nucleic acid molecule associated with the phenotype of the cell, the method comprising:

a) providing a population of cells comprising modifications in a nucleic acid sequence of interest;
b) observing a phenotype of individual cells, wherein the cells are fixed to a surface before, during or after the phenotypic observation, such that the nucleic acid sequence is also immobilised;

c) sequencing the nucleic acid sequence by the method of sequencing according to the description herein, such that an observed phenotype/function of the cell is linked to the nucleic acid sequence.

The population of cell may comprise nucleic acid sequence from a randomised library of nucleic acid modifications. The nucleic acid sequence may be a gene sequence that is modified. The library may comprise 4^N randomized sequence members (e.g. 4^5). The cells may be modified by transformation of a plasmid or nucleic acid fragment library of the modified nucleic acid sequence. Transformation of the plasmids/nucleic acid fragments may provide the population of cells, with each cell making one of many permutations of a certain protein according to the introduced 4^N randomised sequences.

The observed phenotype may comprise any of cell survival, cell killing (such as in the presence of a toxin or antibiotic), cell growth, cell activity, cell membrane transport, cell motility, chemotaxis, cell morphology, cell biochemistry, cell protein expression, cell regulation, cell surface display, cell immunogenicity, cell division; or combinations thereof.

The population of cells may comprise prokaryote or eukaryote cells. In one embodiment, the cells are prokaryote, such as *E. coli*.

The cell may be chemically fixed (e.g., using 1-4% paraformaldehyde), permeabilised (e.g., using treatment with ethanol) to allow access of the sequencing reagents to the nucleic acid of the cell, and then attached to the surface (e.g., to a polylysine-coated surface or to an agarose pad). Alternatively, the cells can be immobilised by capture in microfluidic channels.

According to another description, there is provided a nucleic acid molecule immobilised on a surface, wherein the immobilised nucleic acid molecule is partially duplexed such that it comprises a single-stranded gap-section flanked by duplex sections, and wherein the immobilised nucleic acid is fluorescently labelled.

The immobilised nucleic acid may comprise features as described herein according to the method of the description. In one embodiment, the immobilised nucleic acid molecule is immobilised on a glass surface. The gaps section may be 1, 2, 3, 4, 5, 6, 7, 8, 9, 10, 11, 12, 13, 14, 15, 16, 17, 18, 19, or 20 nucleotides in length. The gap section may be 20 nucleotides in length or less. In another embodiment, the gap section may be 15 nucleotides in length or less. In another embodiment, the gap section may be 12 nucleotides in length or less. In another embodiment, the gap section may be 10 nucleotides in length or less. In another embodiment, the gap section may be 9 nucleotides in length or less. In another embodiment, the gap section may be 6 nucleotides in length or less. In another embodiment, the gap section may be 5 nucleotides in length or less. The immobilised nucleic acid may comprise known/pre-determined sequence and unknown or randomised sequence. The unknown or randomised sequences may be located with the gap section (nonduplexed section).

The immobilised nucleic acid may be isolated (e.g. immobilised apart from other nucleic acid molecules).

According to another description, there is provided an array of surface-immobilised nucleic acids according to the description herein. The array may be arranged in a regular pattern or may be irregular. The immobilised nucleic acid may be spatially separated from each other.

According to another description, there is provided a composition comprising a set of at least four fluorescently labelled oligonucleotide probes, and each probe comprising:

(i) a nucleotide X at one position in the sequence of the fluorescently labelled oligonucleotide probes, wherein the position is the same for each of the at least four fluorescently labelled oligonucleotide probes, and
(ii) universal or degenerate bases at the remaining nucleotide positions,

wherein the at least four fluorescently labelled oligonucleotide probes comprise:

- a first fluorescently labelled oligonucleotide probe wherein nucleotide X is a cytosine;
- a second fluorescently labelled oligonucleotide probe wherein nucleotide X is a guanine;
- a third fluorescently labelled oligonucleotide probe wherein nucleotide X is an adenine; and
- a fourth fluorescently labelled oligonucleotide probe wherein nucleotide X is a thymine or uracil.

The degenerate bases may form a randomised nucleic acid sequence. For example, the degenerate bases may not form a predetermined sequence.

The fluorescently labelled oligonucleotide probes may comprise features as described herein according to the method of the description. The fluorescently labelled oligonucleotide probes may comprise a second fluorophore label. Two fluorophores of a fluorescently labelled oligonucleotide probe may be a FRET pair. The fluorescently labelled oligonucleotide probes may be single stranded, such as ssDNA or analogues thereof.

According to another description, there is provided a fluorescently labelled oligonucleotide probe comprising:

(i) a nucleotide X at one position in the sequence of the fluorescently labelled oligonucleotide probe, wherein X is cytosine, guanine, adenine, thymine or uracil, and

(ii) universal or degenerate bases at the remaining nucleotide positions.

The fluorescently labelled oligonucleotide probe may comprise features as described herein according to the method of the description. The fluorescently labelled oligonucleotide probe may comprise a second fluorophore label. Two fluorophores of a fluorescently labelled oligonucleotide probe may be a FRET pair.

The term "single-molecule phenotyping" used herein is understood to mean the analysis or measurement of an observable characteristic or trait of a single molecule, such as the ability of a single nucleic acid molecule to bind or process or to be processed by another molecule, such as a polypeptide.

The term "highest level of transient binding" used herein is understood to mean the highest overall time spent in the bound state for the seal DNA provided.

The skilled person will understand that optional features of one embodiment or aspect of the description may be applicable, where appropriate, to other embodiments or aspects.

Embodiments of the invention will now be described in more detail, by way of example only, with reference to the accompanying drawings.

**Figure 1.** Single-molecule phenotyping for DNA libraries.

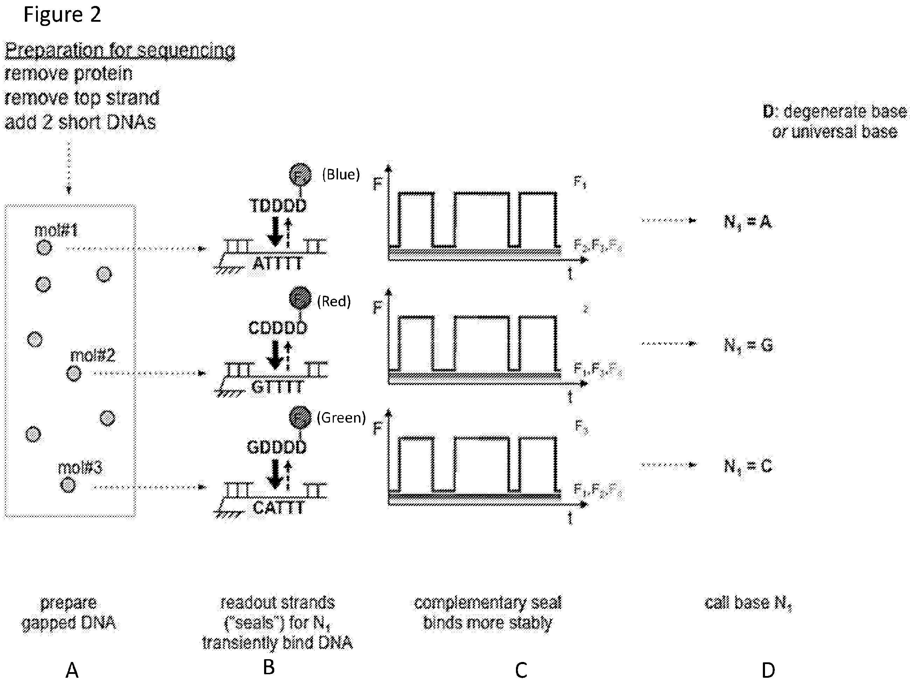

**Figure 2.** Single-molecule gap sequencing: reading base $N_1$.

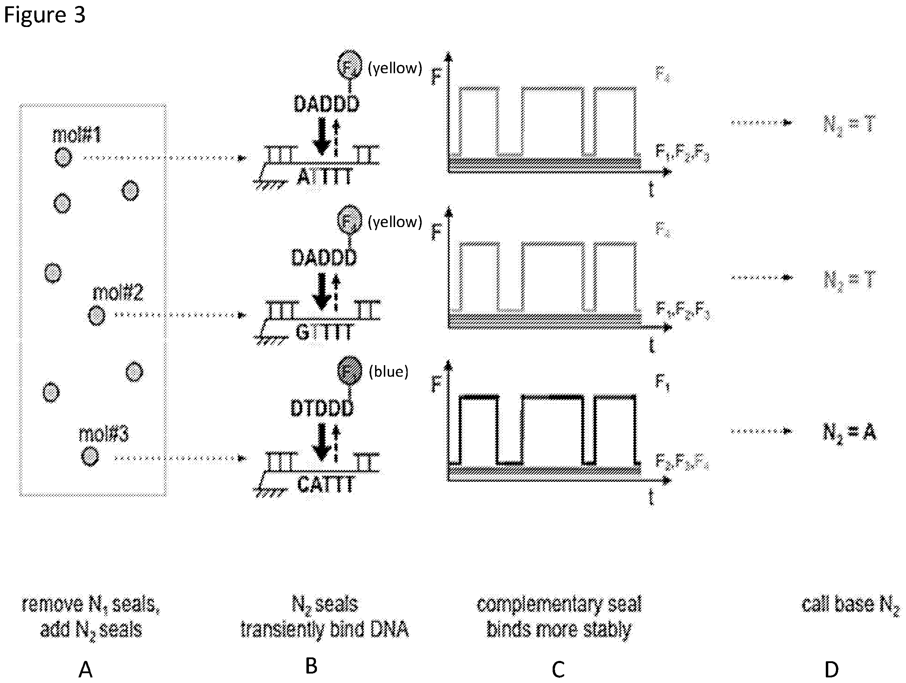

**Figure 3.** Single-molecule gap sequencing: reading base $N_2$.

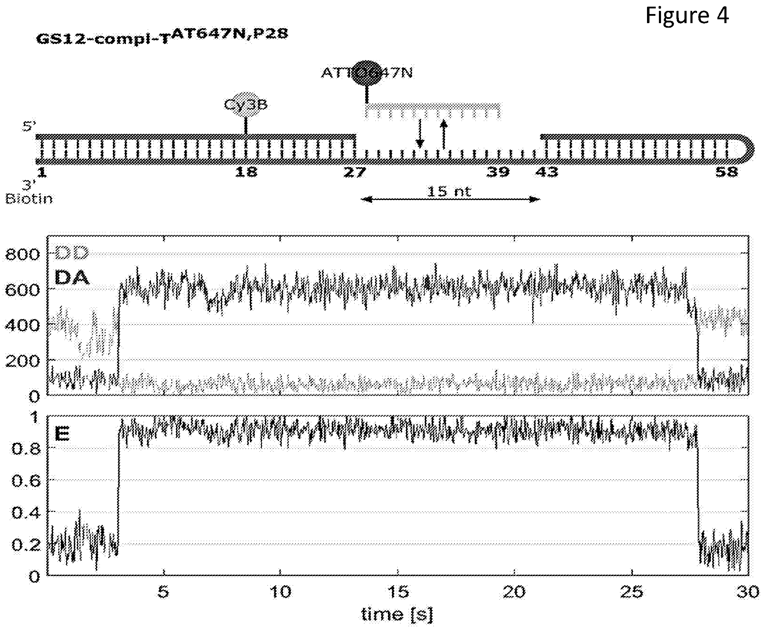

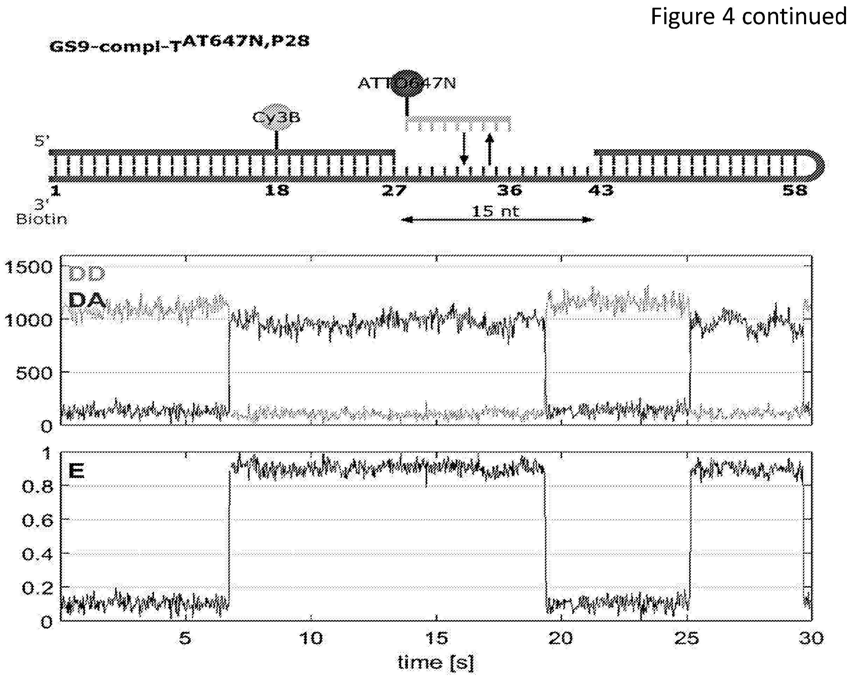

**Figure 4.** Transient binding of GS12-compl-T$^{ATTO647N,P28}$ and GS9-compl-T$^{ATTO647N,P28}$ to biotinylated hairpin gapped-DNA construct. Top traces: Single fluorescence-time trace of transient binding events. Bottom traces: FRET-time trace with E=0.9 FRET for binding events.

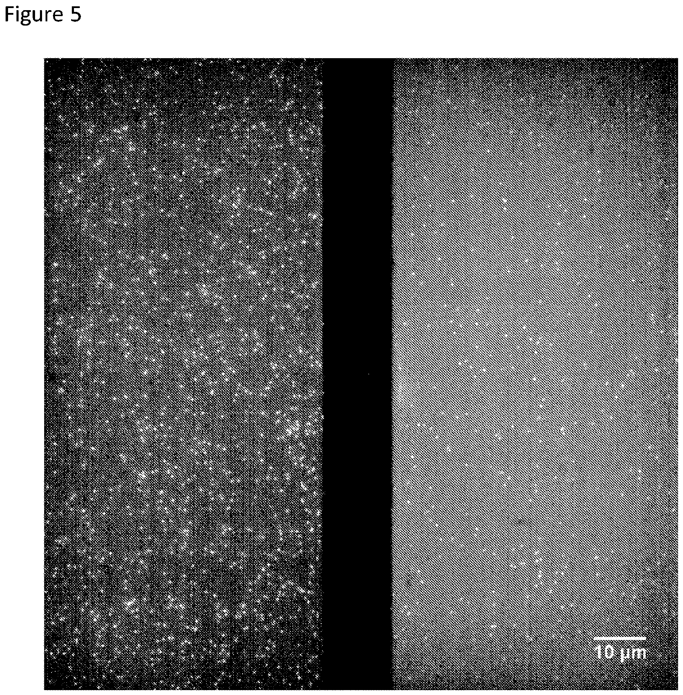

**Figure 5.** Example raw data of biotinylated gapped-DNA constructs immobilised on the surface and 9 nt labelled seals in solution. Left-hand panel (green channel) shows fluorescence from individual Cy3B fluorophores attached to the gapped-DNA constructs when directly excited at 532 nm. Right-hand panel (red channel) shows fluorescence, due to FRET, from individual bound ATTO647N labelled seals.

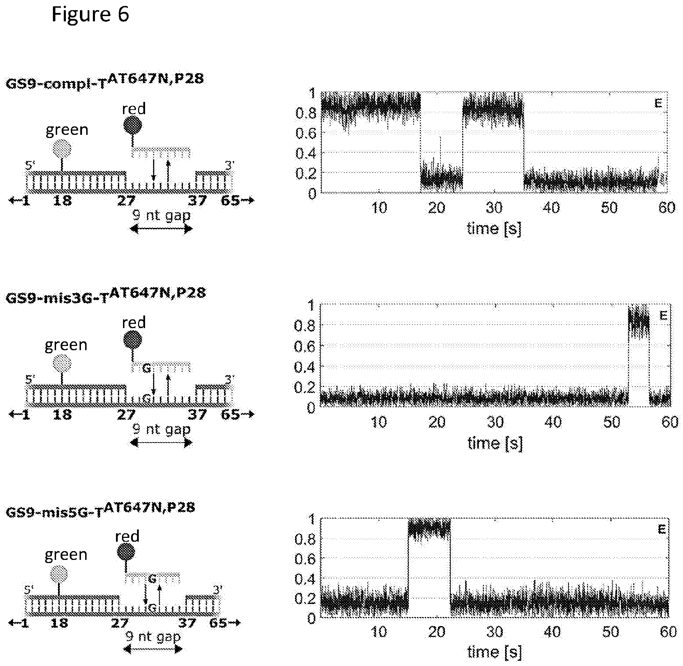

**Figure 6.** Schematics and example FRET-time traces of GS9-compl-T$^{ATTOG47N,P28,}$ GS9-mis3G-T$^{ATTO647N,P28}$ and GS9-misSG-T$^{ATTO647N,P28}$ binding to 9nt gapped-DNA construct.

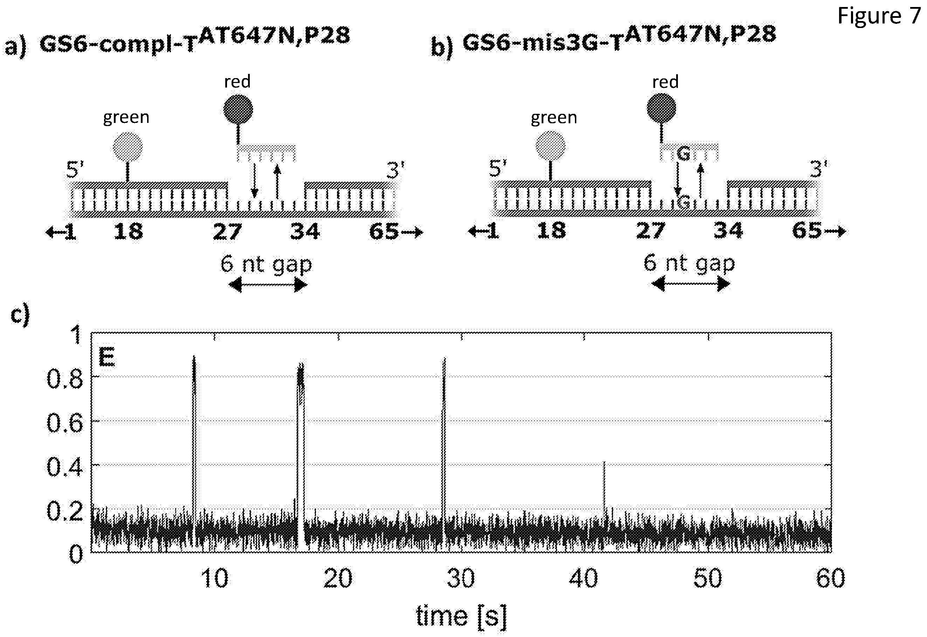

**Figure 7.** a) and b) Schematics of GS6-compl-T$^{ATTO647N,P28}$ and GS6-mis3G-T$^{ATTO647N,P28}$ respectively transiently binding to a 6 nt gapped-DNA construct. c) Example FRET-time trace for 35nM GS6-compl-T$^{ATTO647N,P28}$ showing multiple binding events with a bound time of <1 second.

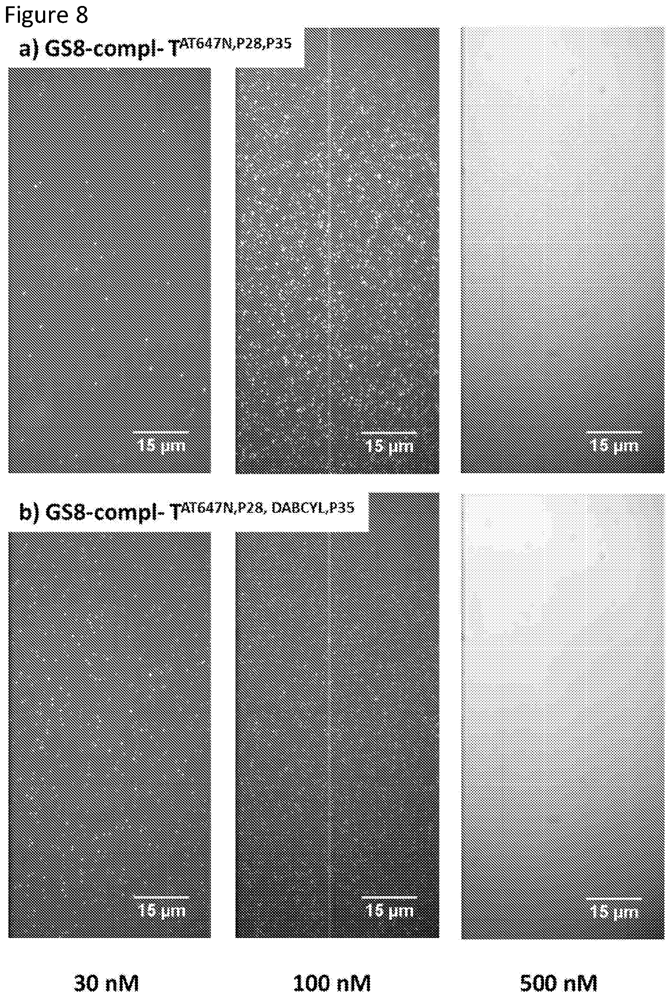

**Figure 8.** Raw microscope data (red channel) of seals and surface immobilised 8 nt gapped-DNA construct and illuminated at 638 nm. a) GSeq-8compl-T$^{ATTO647N\ P28,P35}$ at 30 nM, 100nM and 500 nM. b) GSeq-8compl-T$^{ATTO647N\ P28,DABCYL,P35}$ at 30 nM, 100nM and 500 nM.

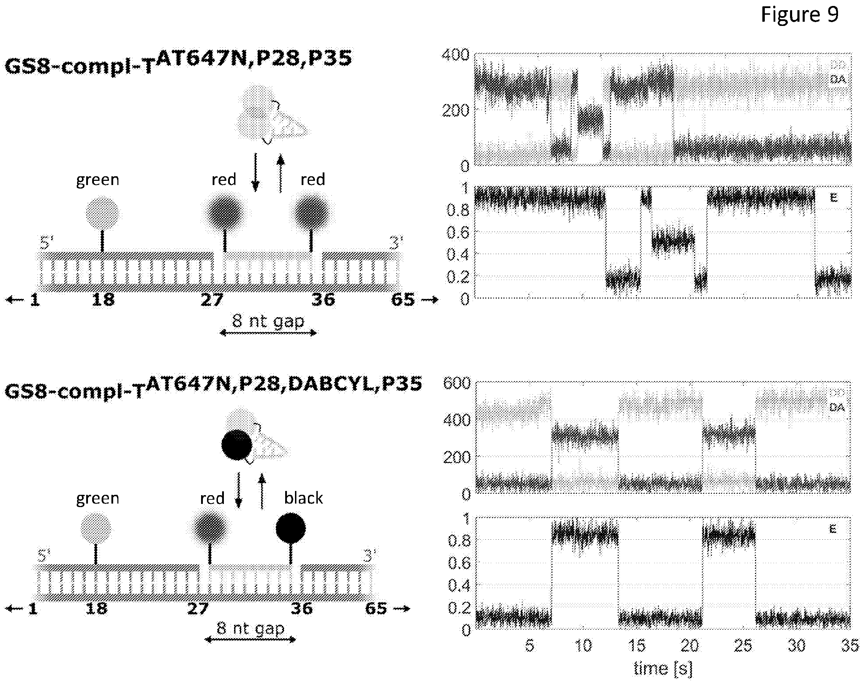

**Figure 9.** Schematics of the fluorescence quenching of GSeq-8compl- T$^{ATTO647N\ P28,P35}$ and GSeq-8compl-T$^{ATTO647N\ P28,\ DABCYL\ P35}$ in solution and un-quenching upon binding. Fluorescence and FRET time traces of 100nM seals transiently binding to a 9 nt gapped-DNA construct. GSeq-8compl-T$^{ATTO64/N\ P28,P35}$ FRET trace shows E=0.5 corresponding to fluorescence from secondary fluorophore only.

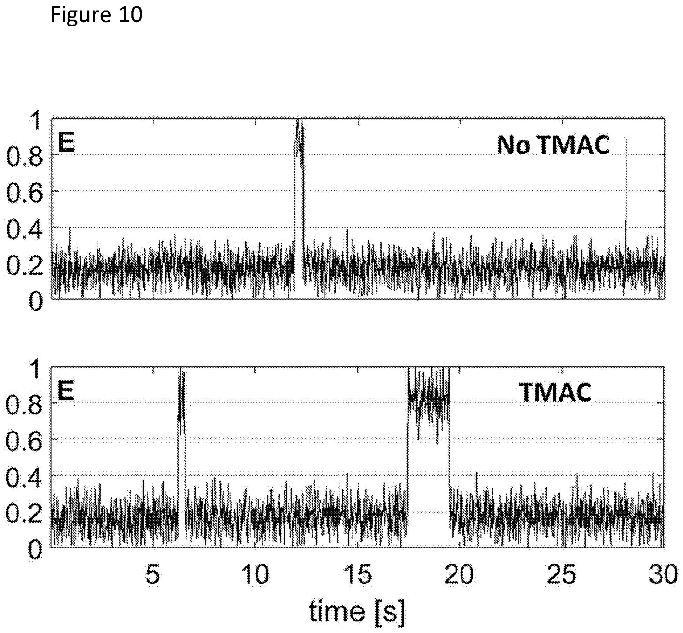

**Figure 10.** Example FRET-time traces of transient binding events of 100nM GS7-ATrich-T$^{ATTO647N,P28,P34}$to a surface immobilised 7 nt gapped-DNA with an A-T exclusive gap in the absence and presence of 3M TMAC. Samples containing TMAC showed an increased bound time.

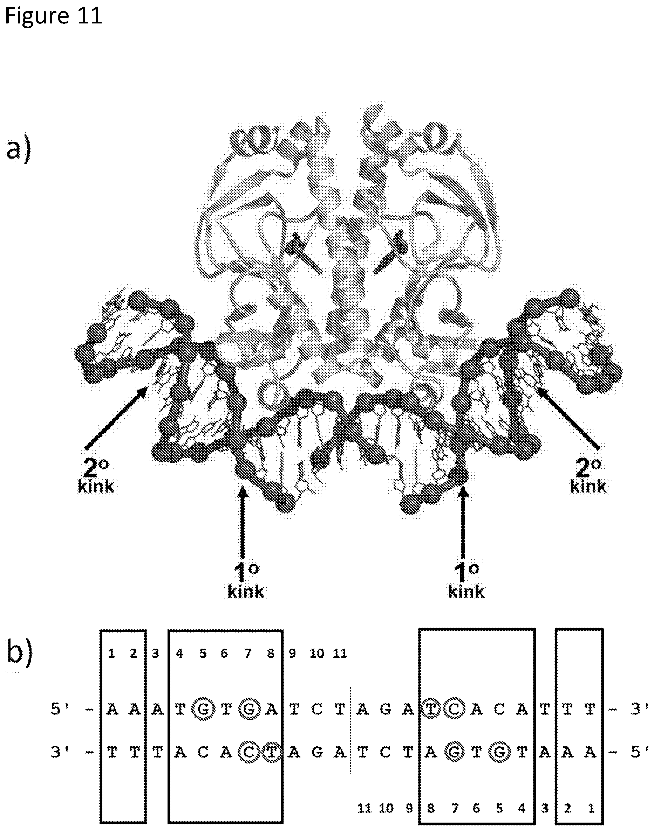

**Figure 11.** a) Structural model of catabolite activator protein (CAP) bound to DNA. CAP bends DNA 80° through primary (1°) and secondary (2°) kinks. b) CAP recognises a two-fold symmetric 22 bp consensus binding sequence. Black boxes indicate the regions of the sequence which highly effect CAP binding, red circles indicate CAP-base contacts. Adapted from [21].

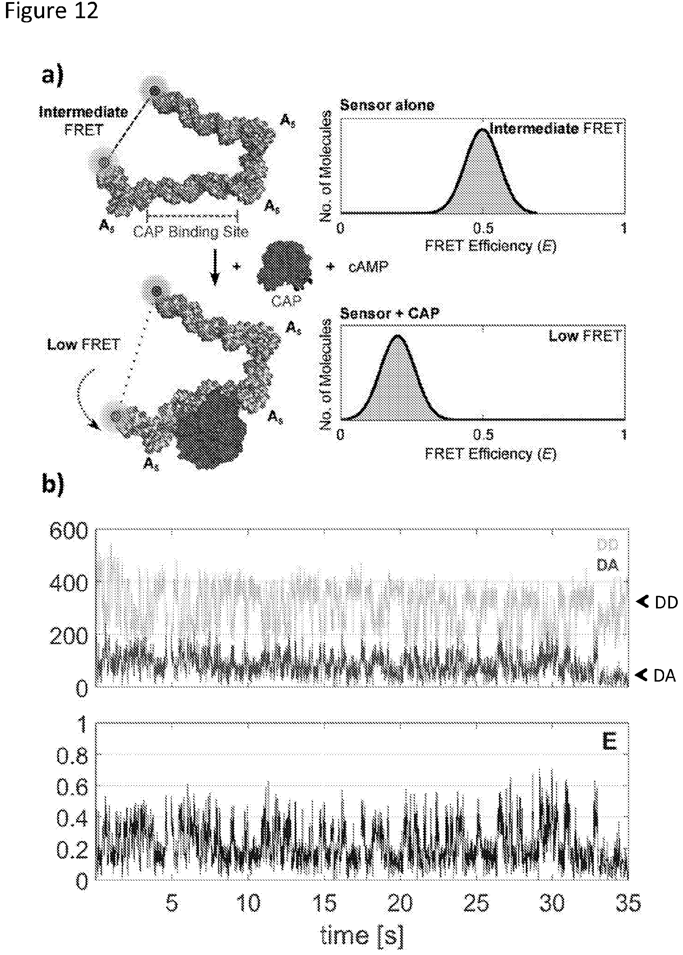

**Figure 12.** a) DNA biosensor has two fluorophores with 0.5 FRET efficiency when illuminated. When CAP binds to the CAP binding site on the DNA biosensor, CAP bends the DNA (increasing the distance between fluorophores) producing a FRET efficiency of 0.2-0.3 [22]. b) Example Fluorescence and FRET time traces showing multiple CAP binding events. FRET efficiency of 0.4-0.5 decreases to 0.1-0.2 upon CAP binding.

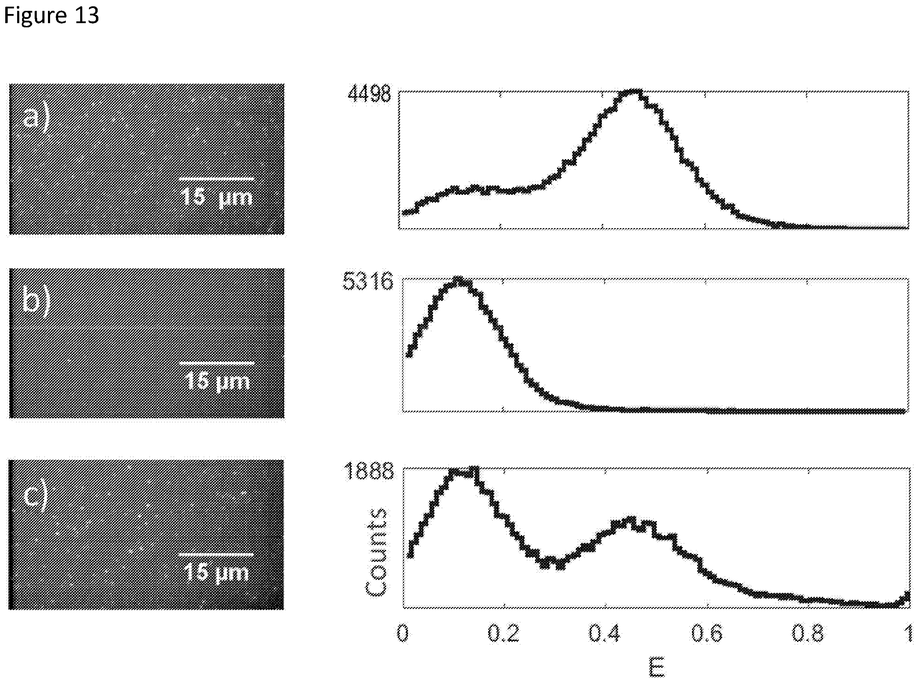

**Figure 13.** a) Surface immobilised DNA FRET standards before NaOH wash. Cropped raw image of red channel of the fluorescence of doubly labelled DNA FRET standards (N=370). FRET distribution shows a majority distribution at E=0.45. b) After 1 minute 10mM NaOH only 2 DNA FRET standards remained. FRET distribution shows a single peak at 0.15 due to noise. c) After re-annealing with 50nM STD45-T$^{AT647N,P28}$ doubly labelled FRET standards are observed (N=136) and a FRET efficiency distribution at 0.45 is recorded. All samples illuminated at 532 nm.

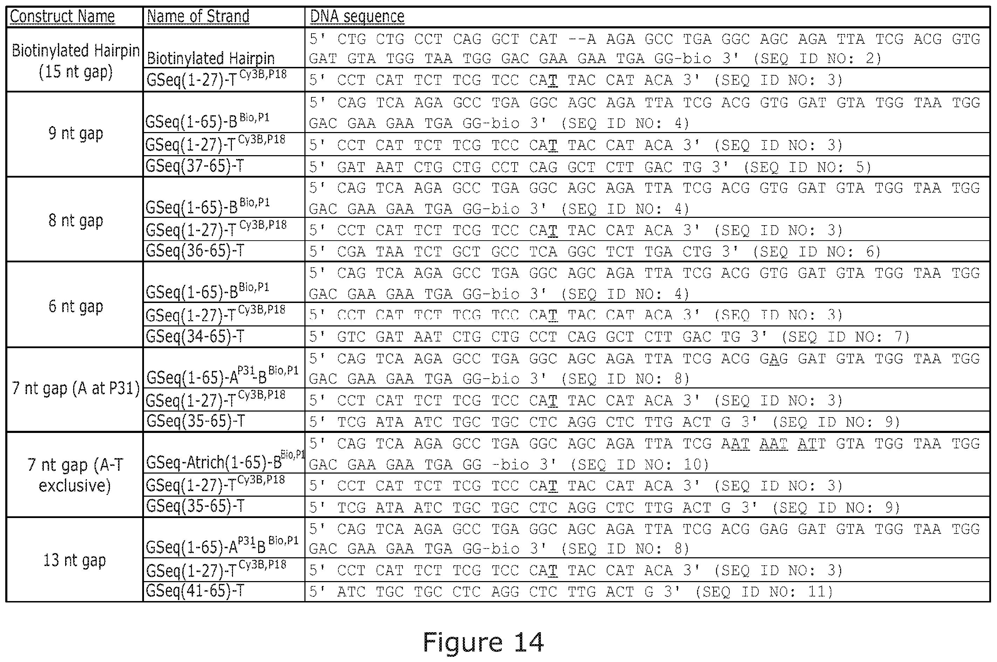

**Figure 14.** Gapped-DNA constructs sequences (5'-3'), labelled bases are in bold and underlined.

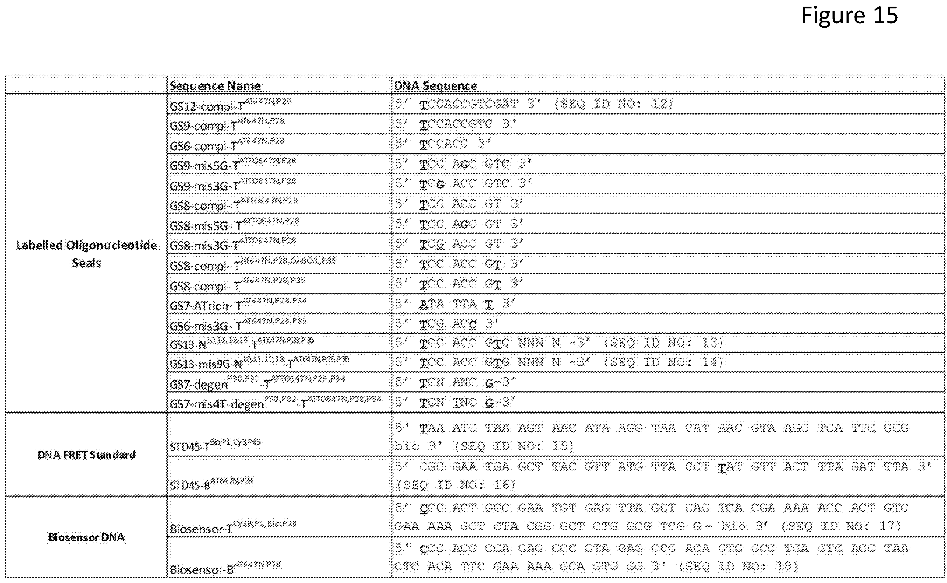

**Figure 15.** Seal DNA sequences, DNA FRET standard sequences and Biosensor DNA sequences (5'-3'), labelled bases are in bold and underlined.

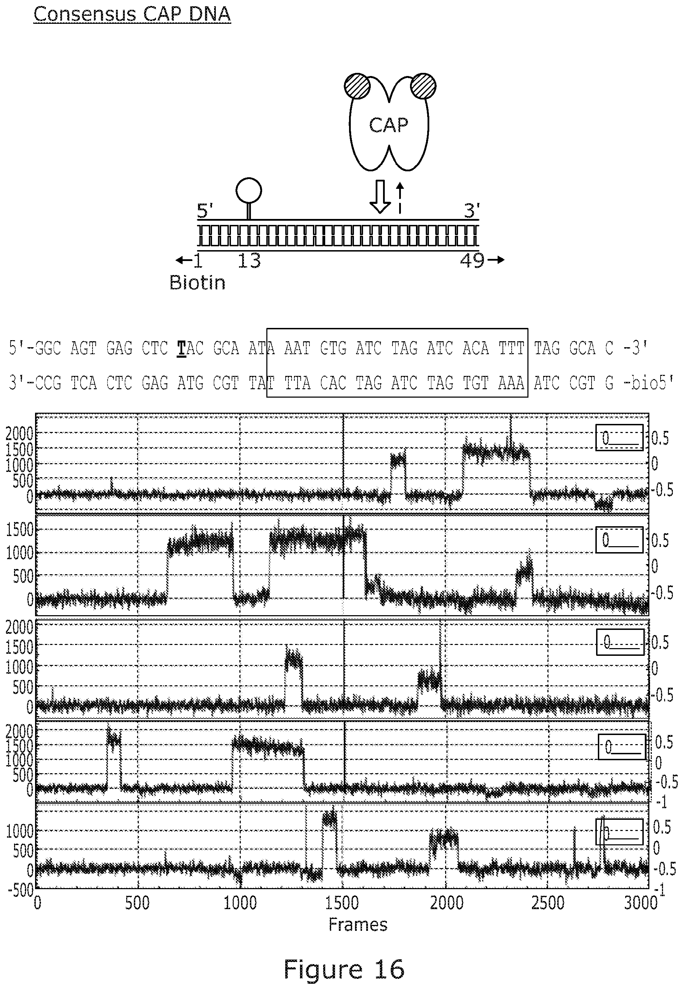

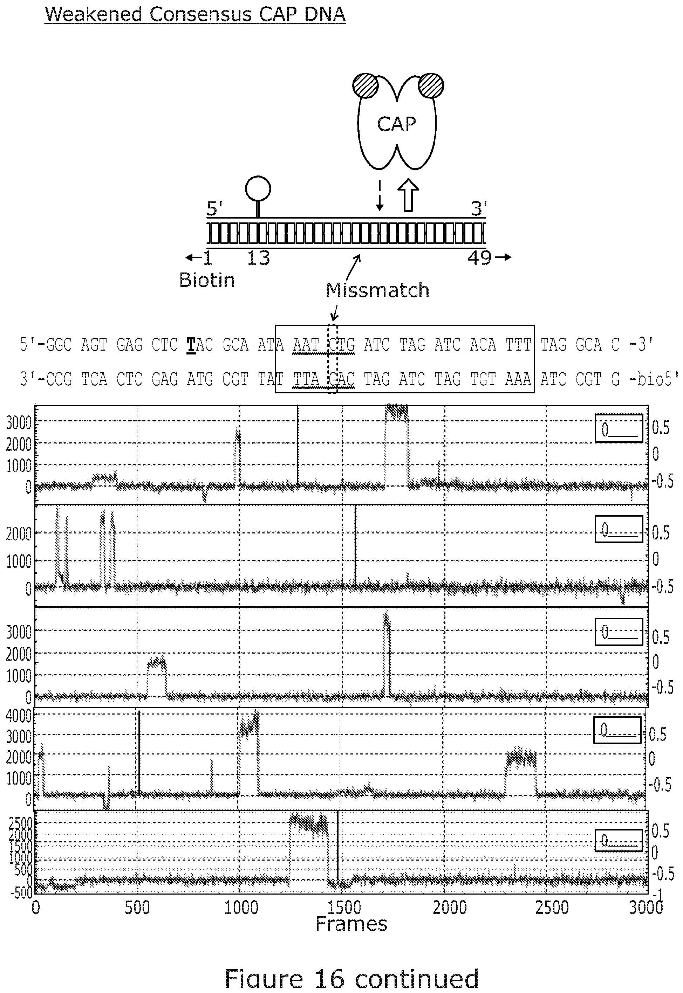

**Figure 16.** Labelled CAP (1.5nM) binding to dsDNA in CAP binding buffer (40 mM Tris-HCl (pH 8.0), 100 mM KCl, 10 mM MgCl2, 5% glycerol, 40% glucose, 0.2 mM cAMP, 1mM Trolox, 40 µg/ml catalase, 0.1 mg/ml glucose oxidase). DNA labelled at position 13 with Cy3B (532 nm excitation) for localisation. Alexa647 on CAP directly excited with 638 nm for single-molecule traces. Sequence of double stranded DNA (yellow region is CAP binding region). Time traces of single-molecule CAP binding (3000 frames = 5 mins) with an exposure time of 100 ms. Lower figures are for a single base modification (position 5 in CAP binding region - red) in the CAP binding region of the dsDNA named 'Weakened Consensus'.

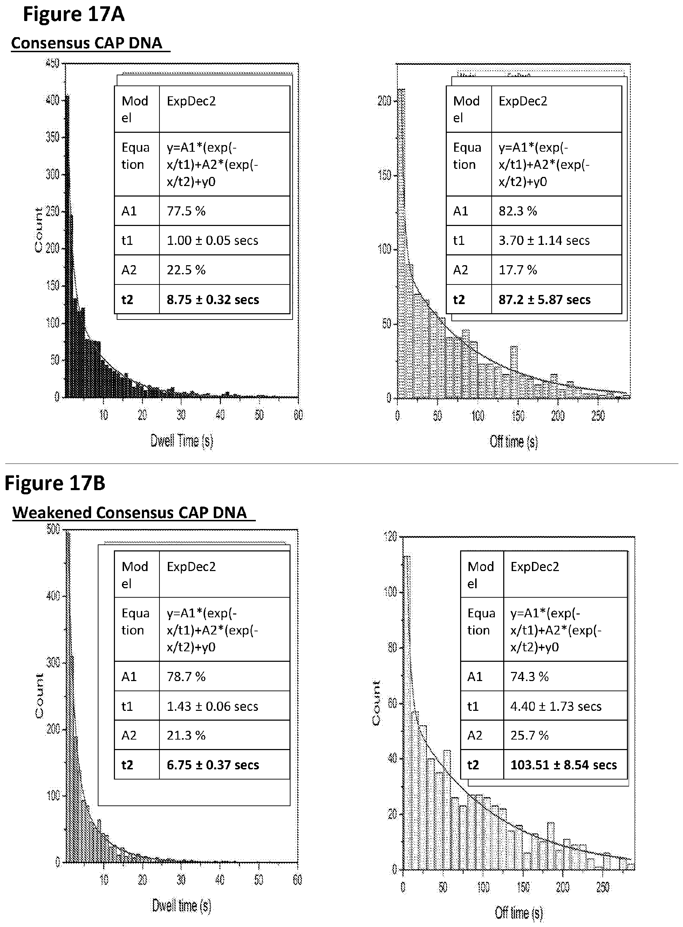

**Figure 17.** Histograms of the CAP dwell time (off rate) and Off time (on rate) for the consensus CAP DNA and the single base modification DNA. 't2' gives the dwell times for comparison. Consensus DNA **(Figure 17A)** gives CAP dwell time of 8.75 ± 0.32 secs, whereas modified DNA **(Figure 17B)** gives CAP dwell time of 6.75 ± 0.37 secs. There is a small difference in the average off time between the two samples.

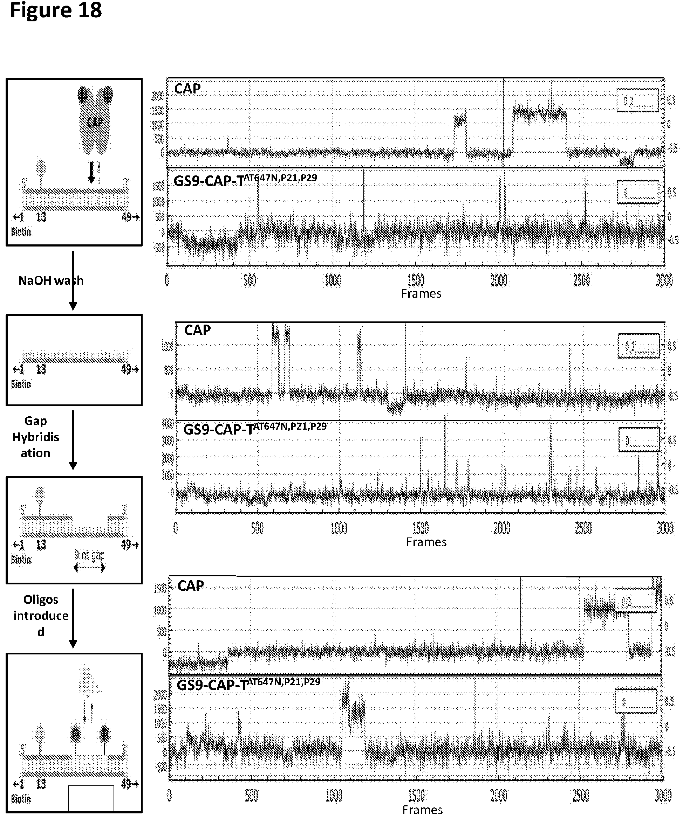

**Figure 18. Single-molecule CAP binding to consensus CAP DNA followed by transient oligo binding.** Left hand column is cartoon of the end to end assay for observing single-molecule protein binding followed by oligo binding on the same DNA. Time traces (3000 frames = 5 mins) for single molecules of CAP (1.5nM) binding to consensus dsDNA. DNA denatured (25 mM NaOH wash for 2 mins) and gapped-DNA formed (mixture of 10 nM GSeq-CAP-(1-20)-TCy3B,P11 and 10 nM GSeq-CAP-(30-49)-T in annealing buffer). Annealing buffer: 20mM Tris-HCl (pH 8.0), 0.5M NaCl, 1mM EDTA (pH 8). Second time trace is of complementary 9 mer oligo (GS9-CAP-TAT647N,P21,P29 100nM) binding in the gapped-DNA of the same molecule.

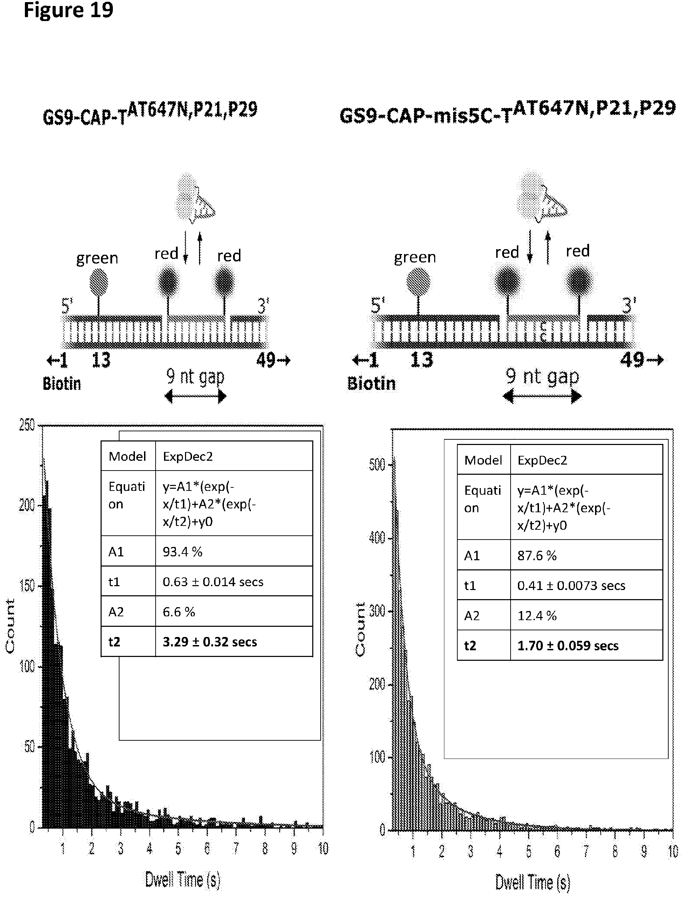

**Figure 19. Dwell time analysis of transient oligo binding in gapped-DNA formed in situ after CAP binding.** Dwell analysis of GS9-CAP-TAT647N,P21,P29 and GS9-CAP-mis5C-TAT647N,P21,P29 binding into gapped-DNA formed using the assay after CAP binding to consensus CAP DNA. The fully complementary oligo gives a dwell time of 3.29 ± 0.32 secs, where as the oligo with a single base-pair mismatch gives a dwell time of 1.70 ± 0.059 secs.

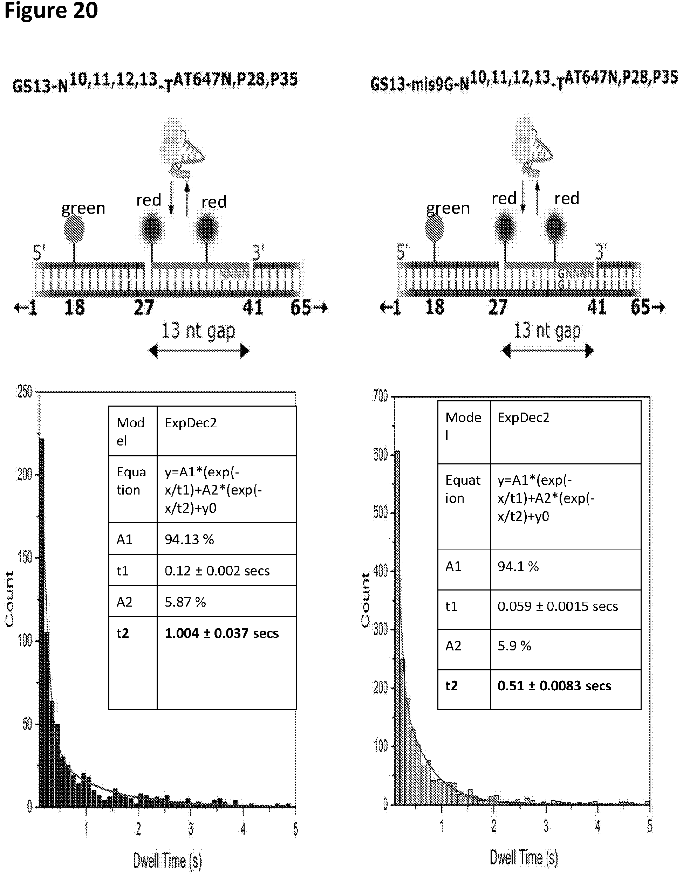

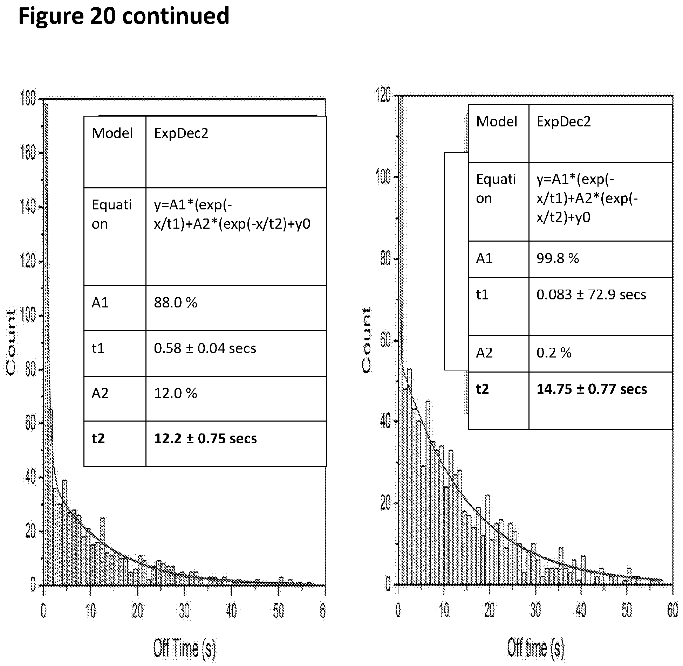

**Figure 20.** Oligos using 5' Nitroindole as a universal base to probe unknown sequences using 10 ms frame rate. The 5 bases at 3' end of oligo will be used to interrogate sequence - one known base and 4 nitroindoles. The dwell time histograms show discrimination between the seal with the complementary known base (dwell time: 1.00 ± 0.04 secs) and the mismatch known base (dwell time: 0.51 ± 0.01 secs) in an imaging buffer containing 0.25 M NaCl. The second line of graphs show that the off time (on rate) also show discrimination all be it smaller.

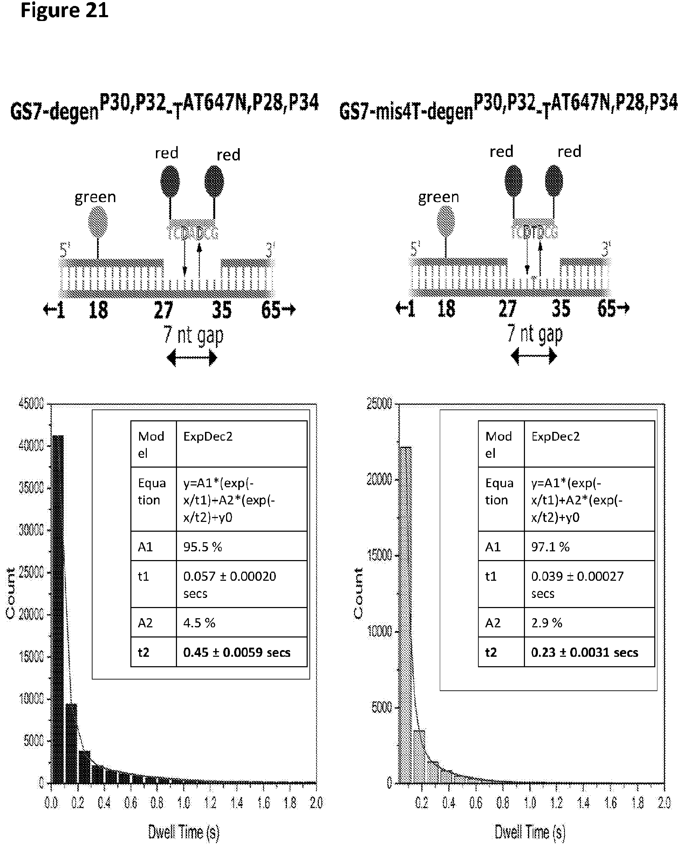

**Figure 21. Dwell time analysis of oligos containing degenerate bases: comparison between fully complementary and a single base-pair mismatch.** Oligos containing degenerate bases in two positions acquired with 10 ms frame rate. This represents having an oligo that can bind to a region of three unknown bases. The dwell time histograms show discrimination between the seal with the complementary known base (dwell time: 0.45 ± 0.0059 secs) and the mismatch known base (dwell time: 0.23 ± 0.0031 secs).

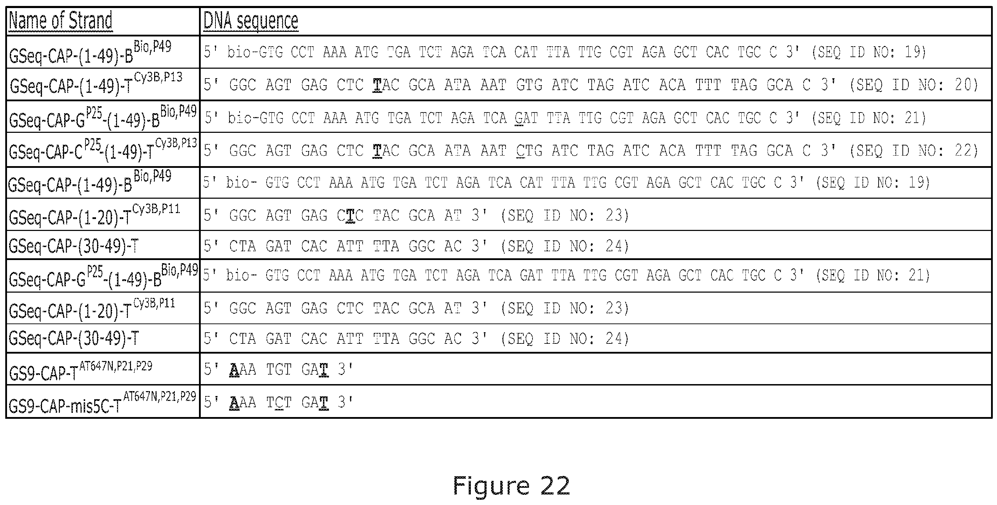

**Figure 22.** CAP DNA sequences (5'-3'), labelled bases are in bold and underlined.

**Gap-Seq: a new single-molecule DNA sequencing method based on transient DNA binding**

**EP 3 682 033 B1**

**Summary**

Single-molecule DNA sequencing methods have made important contributions to genomics, diagnostics and functional analysis of genetic processes. Despite the fact that there are a large variety of single-molecule sequencing methods, there is currently no proficient way to connect the functional properties of a single DNA molecule (single-molecule phenotype) with DNA sequence. This report describes experiments towards the development of 'Gap-Seq', a single-molecule sequencing method that interrogates DNA sequence through the transient or stable binding of short fluorescent DNA strands to surface immobilised gapped-DNA substrates. A base can be identified by the frequency of binding and dwell times of the short DNAs on the immobilised substrates: the correct base shows frequent and relatively long dwells of the short DNAs on the substrates, whereas non-complementary bases show no binding or infrequent, very short dwells on the substrates. The robustness of the technique is tested and strategies to increase the throughput of the method by suppressing the background fluorescence are also employed. Gap-Seq, fills a significant void in single-molecule DNA/RNA sequencing and will be important for both mechanistic work and screening libraries of nucleic acids, peptides and proteins against targets of biomedical importance.

**1. Gap-sequencing: a single-molecule DNA sequencing for single-molecule phenotyping and bar-coding**

The description provides a general, systematic and quantitative way to connect the functionality (reactions, interactions, conformational changes) of single nucleic acids (DNA/RNA) with their sequence. Using large libraries of nucleic acids (such as DNA and RNA oligonucleotides), this capability allows us to identify the sequence-dependence of processes that operate on DNA or RNA substrates, to discover DNA/RNA aptamers for desirable binding/reaction profiles versus various targets (such as proteins, nucleic acids, small molecules etc) and to read bar-codes that can be introduced in biological molecules and cells. The current availability of high-throughput, automated single-molecule imaging enables this unique connection through our assay.

The procedure that links the functionality and sequence at the single-molecule level involves three main steps: the single-molecule phenotyping step; the preparation step, and the sequencing step.

Step **1: single-molecule phenotyping.** With reference to Figure 1, this step involves a functional analysis of libraries of DNA molecules immobilised on a surface, typically a glass coverslip. Initially, a library of nucleic acids (e.g., DNA) is prepared using automated DNA synthesis with degenerate nucleotides inserted at the region of DNA to be interrogated for function. The library has $4^N$ members, where N is the number of randomised bases, e.g., a randomised 5-nucleotide region will be represented by 1024 possible DNA sequences (Fig.1A). Initial uses of the assay will be confined to 9-nt in length, representing libraries of up to $2.6 \times 10^5$ different sequences.

Each DNA has a functionality (such as 5' or 3'-biotinylation of the DNA) that allows its surface-immobilisation on neutravidin-coated glass surfaces. The DNAs are immobilised to allow their detection on the surface at the single-molecule level, and are imaged on a single-molecule fluorescence microscope with a large field-of-view; sCMOS-based wide-field imaging allows parallel detection of 500-2000 single molecules per field-of-view (Fig. 1B); a high-precision localisation version of the assay allow much higher densities to be implemented ($1.5 \times 10^7$ molecules per 120 $\mu$m x120 $\mu$m field of view).

The different DNAs are then interrogated in terms of functionality. The functional assay may be the interaction of the DNA with a sequence-dependent DNA binding protein, such as a transcription factor, or a viral protein; the sequence-dependent processing of the DNA by a nucleic-acid polymerase, such as the RNA polymerase or DNA polymerase; the interaction of the DNA with another nucleic acid, e.g., another DNA or RNA strand; or --in the case that the immobilised nucleic acid is an aptamer-- the target protein, biomolecule or particle.

An example of functional assay can be the binding and subsequent dissociation of a DNA-binding protein. The protein may be fluorescent; in such a case, binding of the protein to the DNA results (Fig. 1C, top and middle) in a clear fluorescence observable, which is repeated binding events of the protein at the same location of the surface (Fig. 1D).

If the binding is strong (Fig. 1C, top), the protein interacts with the DNA for extended times (long $t_{on}$ times; Fig 1D, top panel), and for a given concentration in the 1 nM- 100 nM range, it stays off the DNA only for a short time (short $t_{off}$ times; Fig 1D, top panel). Repeated binding and dissociation events allow the collection of significant statistics from an individual DNA molecule; these statistics will be sufficient to construct dwell-time frequency distributions that in turn can be fit to exponential functions (typically, single-exponential functions) to obtain the rate constants for binding and dissociation, and thus the affinity of the interaction expressed as the equilibrium binding constant ($K_D$) for the single DNA molecule in question. The identification of the base corresponding to fully complementary binding can also be provided by identifying the (color-coded) base that provides the highest fraction of binding-associated frames (vs the other 3 bases); this approach carries similarity to base calling in 4-colour Sanger sequencing via capillary electrophoresis. Since the location of the DNA/RNA molecules on the glass surface is unchanged, there is a direct connection between phenotype and DNA/RNA sequence.

If the binding is weak (Fig. 1C, middle), the protein interacts with the DNA for short times (short $t_{on}$ times; Fig 1D,

14

middle panel), and for a given concentration in the 1 nM-100 nM range, it stays off the DNA for a long time (long $t_{off}$ times; Fig 1D, middle panel). Similar analysis to the strong-binding sequence recovers the rate and thermodynamic constants for the weak-binding sequence. Further, there will be sequence where protein binding is not detected; in such cases, the information obtained from a specific sequence is its absence of binding.

To help the identification of location of DNA molecules, especially when binding is absent, the DNA molecules can be initially labelled by a fluorophore inserted away from the site of interaction of a reaction; this fluorophore can be used to detect the protein binding, or can be used to obtain structural information about the protein-DNA complex; the DNA fluorophore may be removed in subsequent steps. Further, the presence of signal during subsequent sequencing steps will identify their presence. The DNA can also be labelled by one or two fluorophores that can detect the binding of an unlabelled protein either through an environmental change (for the single fluorophore) or conformational change (for the fluorophore pair, e.g., via FRET).

**Step 2: preparation for sequencing.** This simple step removes the interacting molecule (using a simple wash, which can involve conditions that make any binding much weaker, such as a wash of a DNA-binding protein with a high salt buffer). The DNA is then denatured to remove the top strand (i.e., the strand that is not directly linked to the surface); this can be done either chemically (using treatment with high NaOH concentration) or thermally (by melting the strands using hot water). Finally, two shorter DNA strands (gap-flanking strands, GF1 and GF2) are hybridized stably to the surface-immobilised DNA ("template" strand) to create a DNA gap close to the middle of the sequence of the template strand.

**Step 3: gap-sequencing.** With reference to Figure 2, this method depends on evaluating the transient and repeated binding of short fluorescent DNA oligos ("seals"; typically 6-12 nt long) that are used for base readout to the complementary gapped DNAs carrying a short DNA gap with length equal to the seal. This is an important feature, since no other binding registers are possible; the correct, the full complementary register is supported by the highest possible degree of base pairing, plus the full stabilization energy coming from stacking of DNA end on both sides. As a result, fully complementary seals bind more stably and more frequently to the complementary gapped DNA; e.g., after staying on the target for 0.1-10 sec, they dissociate and rebind to the same gap DNA target; the repeated interrogation of the same DNA provides robustness to the detection of the correct base. If the DNA is non-complementary, the binding is very weak or non-existent.

The identification of the correct base is done at a single step with a different set of DNAs that interrogate a single base within the DNA gap. The DNAs can be designed in a way that only the central part of the gap is read, e.g., the central 5 of the 9 nucleotides of the gap (bases 3, 4, 5, 6, 7, corresponding to bases $N_1$, $N_2$, $N_3$, $N_4$ and $N_5$ of the randomised library); bases 1, 2 and 8, 9 are known and their complementary bases are used in the equivalent position on the seal DNA to provide more stable binding and facilitate the design of readout DNA sets. Single mismatches at bases 3, 4, 5, 6, 7 are known to destabilise the binding of 9-mer, allowing clear discrimination between a fully complementary seal DNA and a single-mismatch seal. Bases 1, 2 and 8, 9 can also be randomized, since they are also expected to affect binding due to perturb stacking to the edges of the gap, and to reduced base pairing compared to the fully complementary strand.

The set of readout DNAs for position $N_1$ can constitute be just 4 seal DNAs that are color-coded (e.g., blue, green, orange, and red) in a way that can be easily detected via spectroscopic signatures using a 2-laser, 2-excitation single-molecule fluorescence format. These DNAs differ only in the position complementary to $N_1$ and the colour code: blue for templated A; green for templated C; orange for template C; and red for templated G (Fig. 2B). The remaining four bases ($N_2$-$N_4$) are universal bases (i.e., bases that pair in the same manner with A, C, G, T); such bases include 5-nitro-indole (the best characterised of universal bases) and include several other bases.

The remaining four bases ($N_2$-$N_5$) can also be degenerate bases (i.e., an ensemble mixture that contains 25% of each bases on bases $N_2$-$N_5$); in this case, only the fully complementary seal will bind to the gap; however, in the method that uses degenerate DNAs, only a small fraction (1 out $4^5$ DNAs) provides a binding signal, decreasing the frequency of seal association, and requiring higher concentration of the library DNAs to operate, which in turn increases the background fluorescence of unbound DNAs; note that the limit of operation using total-internal reflection fluorescence microscopy (the preferred way to minimise background in wide-field camera-based detection) is ~50nM.

To allow operation at higher concentration, there are various ways to suppress the fluorescence of unbound molecules. Many of these strategies use fluorescence quenching. A successful strategy involves the attachment of two copies of the same fluorophore (e.g., ATTO647N) to the two end of the DNA seal; when the seal is unbound, and thus in the flexible ssDNA form, the two fluorophores are in close proximity and statically quenched each other, changing their spectroscopic properties and preventing fluorescence emission. However, upon binding, the seal becomes double-stranded, the fluorophores move further away, become dequenched, and the binding can be easily seen (as appearance of the fluorescence equivalent to two ATTO647N fluorophores localising on the interrogated DNA). This strategy allows use to operate with low background at ~ 1μM concentration of unbound fluorescent seal DNAs. Other fluorophores can also be used, allow color-coding of the quenched seal DNAs.

To read base $N_2$, the set of seals for $N_1$ is washed away, and a different set of readout probes is introduced. The new set has different, color-coded bases in the position complementary to $N_2$ (see Fig 3B), and the rest of the bases are universal (or degenerate, if the 2nd strategy for ensuring binding is used). Similar analysis to $N_1$ identifies base $N_2$, and the

addition of different sets of seals completes the sequencing of position $N_3$, $N_4$ and $N_5$.

In conclusion, a combination of 1 wash and 1 incubation per interrogated bases should be sufficient to call a base. The envisioned timescale for a single-base reading for a field-of-view (~1,000 molecules) is 1 min. For the super-resolution imaging mode, more time will be needed per field-of-view, but just one or very few FOVs will be sufficient for covering large libraries of interrogated molecules.

Another way to increase the speed of the assay and decrease the concentration of fluorescent seal DNAs needed is to amplify the gaps region via rolling circle amplification of the DNA template that has been used for the functional assay. If the DNA used in the functional assay is linear, then the non-surface-immobilised strand will be removed, and the surface-immobilised strand will be circularised using a splint DNA to bring close the DNA ends, followed by ligation. The splint DNA can also be used as a primer for RCA, which can produce >100 copies of the randomised sequences within an hour (Mohsen and Coll, Acc Chem Res, 2016); removal of the DNA polymerase and annealing of single gap-flanking oligo (which essentially combines the two gap-flanking DNAs) provides >100 gaps in the amplified but still surface-immobilised DNA, maintaining the link between the initial phenotype and the sequence. The abundance of targets (>100) will decrease the time needed for binding or the concentration of fluorescence seals needed for binding by a similar fold (~100-fold).

Strategy to read the sequence along with the entire gap of 9 nt use 9 sets of 9-mers (universal or degenerate). Beyond the length of 9 nt, strategies to move the gap along the DNA strand are also possible, increasing the length of the read sequences to 20 or more bases. This is an important length for screening aptamer DNA/RNA libraries against a large number of targets. A way to walk the gap (after the reading of the first 5 bases within the 9-nt gap) is to use annealing of a left gap-flanking DNA that contains a mix of universal bases (that covers the randomised region) and specific bases (that covers the known region, i.e., positions 8 and 9 in the initial gap, and provides stable binding and register); on the other hand, the right gap-flanking oligo will be 9 base-pairs shorter (while during the initial round, it was designed similarly to how the left gap-flanking DNA was designed for the second round). To look at the effect of the initial positions 1, 2, 8, and 9, they can be randomised using a different set of oligos, and the results from both libraries can be combined in a way that provides the effect of a more extensive sequence context.

## 2 Methods **and** Experimental Procedures

### 2.1 DNAs

A table of the gapped-DNA construct's sequence is included in Fig. 14. All DNA constructs were annealed in equimolar amounts in annealing buffer (200mM Tris pH 8, 500mM NaCl and 1mM EDTA) by heating to 90°C and slow cooling to 4°C.

The sequences for the seals (also known as fluorescently labeled oligonucleotide probes), DNA FRET standard and DNA biosensor are documented in Fig. 15. DNA FRET standard and DNA biosensor were annealed using the same method for the gapped-DNA constructs. STD45-T[AT647N,P28] was prepared through NHS-ester labelling of the amine-modified STD45-B with ATTO647N following established protocols [12]. Also described is nucleic acid comprising or consisting of sequences described herein, or compositions comprising such nucleic acid, or combinations thereof.

Explanation of the nomenclature - an example strand is GSeq(1-27)-T[Cy3B,P18] GSeq refers to the gap sequencing experiments, (1-27) documents the length of the strand and the position within the construct with respect to the 5' end, T represents top strand and [Cy3B, P18] notes the position of modifications.

The Biotinylated Hairpin, GSeq(1-27)-T[Cy3B,P18], GS12-compl-T[AT647N,P28], GS9-compl-T[AT647N,P28], GS6-compl-T[AT647N,P28], STD45-B[Bio,P1,Cy3,P45] and STD45-T were purchased from iba. Biosensor-T[Cy3B,P1,Bio,P78] and Biosensor-B[AT647N,P78] were previously ordered from Integrated DNA Technologies. The biotinylated DNA constructs were immobilised on polyethylene glycol coated coverslips [13].

### 2.2 Buffers

Imaging buffer: NaCl 0.5 M, HEPES 50mM, BSA 6mM, Trolox 1mM and seals at specified concentration. The imaging buffer also contained an oxygen scavenging system: 1% Glucose, 40 $\mu$g ml$^{-1}$ and 0.2 mg ml$^{-1}$ glucose oxidase.

CAP Imaging: 600 nM CAP, 0.2 mM cAMP, 1 mM Trolox, 1xPBS, 1% Glucose, 40 g ml$^{-1}$ and 0.2 mg ml$^{-1}$ glucose oxidase.

### 2.3 In situ denaturing and re-annealing of double stranded DNA

The DNA FRET standards were initially imaged in the presence of imaging buffer. The sample was then washed three times with PBS 1x. After washing the sample was incubated with 10mM of NaOH for one minute. The sample was then washed again with PBS 1x before being imaged in imaging buffer. After imaging, the sample was washed again with PBS 1x followed by an 1 minute incubation of 50nM STD45-T[AT647N,P28]. The sample was then washed again with PBS 1x and imaged in the presence of imaging buffer.

**2.4 Förster resonance energy transfer (FRET)**

The fluorophores Cy3B and ATTO647N are commonly used together as a donor and an acceptor pair for Förster resonance energy transfer (FRET). FRET occurs when energy is transferred through dipole-dipole interaction between an excited donor molecule and an unexcited acceptor molecule. The energy transferred between the two molecules is non-radiative and heavily distance dependent. The FRET efficiency can be calculated using the following equation:

$$E = \frac{1}{1 + (R/R_0)^6} \qquad (1)$$

where R is the distance between the two dipoles and R0 is the Förster radius (R where the E=0.5). E* denotes the apparent FRET efficiency, which is commonly used to quantify changes in fluorescence intensity, is defined as:

$$E^* = \frac{F_{D_{exc}}^{A_{em}}}{F_{D_{exc}}^{A_{em}} + F_{D_{exc}}^{D_{em}}} \qquad (2)$$

where F $A_{em}/D_{exc}$ is the fluorescence intensity of the acceptor when the donor is excited and F $D_{em}/D_{exc}$ is the fluorescence intensity of the donor when it is directly excited [14]. E* is used throughout this report as the measure of FRET efficiency.

**2.5 Microscopy and Data Analysis**

Movies were collected using the Oxford Nanoimager microscope [15] at a maximum frequency of 100 Hz. The microscope was used as a widefield single-molecule fluorescence microscope with objective based total internal reflection illumination at 52°. Continuous wave excitation lasers (532nm and 638 nm) were used to excite ATTO647N and Cy3B/Cy3 respectively with the laser power adjusted to the experiment. The data was analysed using single-molecule FRET analysis software from Oxford Nanoimaging.

**3.1 Development of Gap-Seq (Step 3)**

**3.1.1 Observing Transient Binding**

The first experiments conducted aimed to observe transient DNA binding to surface immobilize biotinylated gapped-DNA hairpin constructs, labelled with Cy3B. The DNA gap was 15 nt long with individual experiments conducted for complementary ATTO647N labelled seals of different lengths in solution. Seals were designed to bind immediately at the 5' end of the gap with a 10-bp separation between fluorophores resulting in a theoretical FRET efficiency of 0.9 (Fig. 4).

Individual experiments were conducted for GS12-compl-T$^{ATTO647N,P28}$, GS9-compl-T$^{ATT0647N,P28}$ and GS6-compl-T$^{ATTO647N,P28}$. Under 532nm illumination, a binding event was characterised by co-localised fluorescent spots in both channels of the raw data (Fig.5). Transient binding events were observed for 75nM GS12-compl-T$^{ATTO647N,P28}$ and 30nM GS9-compl-T$^{ATTO647N,P28}$ (Fig. 4). A sharp increase in red fluorescence signified a binding event due to ATTO647N being excited by the evanescent laser wave. The fluorescence intensity time traces show an anti-correlation between the fluorescence observed from Cy3B and ATTO647N, corresponding to a FRET efficiency of 0.9. No transient binding events were recorded for GS6-compl-T$^{ATTO647N,P28}$

The experiments showed successful observation of repeated transient binding of 12 nt and 9 nt seals, within the 15 nt gapped-DNA, with seals of 6 nt thought to dissociate too quickly to be detected under these conditions.

**3.1.2 Detecting a single base pair mismatch**

To create an adjustable sized DNA gap, the hairpin was redesigned as two separate strands, a biotinylated bottom strand and an unlabelled fully complementary top strand (right flanking) with the left flanking labelled top strand remaining the same (Fig. 6). The selection of the length of the right flanking top strand allowed the size of the gap to be altered. The whole construct was lengthened to 65 bps by the addition of 5 bases at the 3' end to represent the length of DNAs that will be sequenced with Gap-Seq. Seals were designed to be shorter in length for faster repeated binding as previously GS12-compl-T$^{ATTO647N,P28}$ bound too stably.

The main aim of the second round of experiments was to discriminate between fully complementary seals and seals with a single base pair (bp) mismatch. Single internal mismatches were created in the seals by substituting cytosine for guanine, one seal with a position 3 mismatch and another with a position 5 mismatch. The length of an oligo [16] and the number of consecutive complementary bps [17] determine the bound time. Therefore, a seal with a single bp mismatch would be expected to bind for a shorter period of time than a fully complementary seal, especially if the mismatch breaks a large number of consecutive bases. A strong bp was chosen to be mismatched to heighten the increase in the dissociation rate.

The first single bp mismatch experiments were conducted with 30nM of 9 nucleotide seals: GS9-compl-TATTO647N,P28 (fully complementary), GS9-mis3G-TATTO647N,P28 (mismatch-position 3) and GS9-mis5G- T$^{ATTO647N,P28}$ (mismatch-position 5) binding to a 9nt gapped-DNA construct.

Transient binding events were observed for all the seals (Fig. 6). GS9-compl-T$^{ATT0647N,P28}$ bound with an average dwell time of 12±0.5 seconds, GS9-mis3G-T $^{ATTO647N,P28}$ with an average dwell time of 7±0.5 seconds and GS9-mis5G-T$^{ATTO647N,P28}$ bound with an average dwell time of 6±0.5 seconds. Bound time distribution of the seals could not be calculated due to software limitations. The difference in the stability of the binding allows discrimination between the fully complementary seal GS9-compl-T$^{ATT0647N,P28}$ and those with a single bp mismatch, showing that a single bp mismatch could be used to read a single base.

Single bp mismatch experiments were also conducted with a 8 nt gapped-DNA construct and 30nM 8 nt seals: GS8-compl-T$^{ATTO647N,P28,}$ GS8-mis5G-T$^{ATTO647N,P28}$ and GS8-mis3G-T$^{ATTO647N,P28}$ Transient binding events were observed for all the 8 nt probes, with shorter bound times observed for the mismatch seals.

The experiments documented above have shown that there is a clear difference in the binding kinetics of fully complementary seals and seals with a single bp mismatch, demonstrating a possible way to sequence a single base.

### 3.1.3 Binding of 6 nt seal and single bp mismatch

Shorter seals are desirable due to the shorter bound time to the gapped-DNA leading to a faster interrogation of the sequence. Previously the 6nt seal GS6-compl-T$^{ATTO647N,P28}$ was not observed to bind with the biotinylated hairpin gapped-DNA construct (15 nt gap). Experiments were conducted again with GS6-compl-T$^{ATTO647N,P28}$ and a single bp mismatch 6nt seal GS6-mis3G-T$^{ATTO647N,P28,P33}$ with a 6 nt gapped-DNA construct (Fig.7 a & b).

Transient binding events on the order of 100-500 ms were observed for 35nM GS6-compl-T$^{ATTO647N,P28}$ (Fig.7 c) showing that the binding of a complementary 6 nt seal can be observed when stabilised by binding to a 6 nt gapped-DNA. However, no transient binding events were detected for 100nM GS6-mis3G-T$^{ATTO647N,P28,P33}$ allowing discrimination between a fully complementary seal and a mismatch seal due to the limit of detection for the microscope (100 Hz).

These experiments have shown 6-nt seals to be the shortest possible seals that can currently be used with a clear distinction between fully complementary and a single bp mismatch seals. Seals of 5-nt in length may also be captured if faster/longer acquisition is used under conditions using higher salt concentrations (3M NaCl).

### 3.1.4 Fluorescence Quenchers

The initial experiments conducted were dependent on FRET due to limitations with data analysis, however, FRET is not necessary for the Gap-Seq method. Binding events can be identified by the increase in fluorescence from the seal alone, with the gapped-DNA construct labelled to allow co-localisation. Therefore, the excitation wavelength would be chosen to directly excite the fluorophore attached to the seal, for example, 638nm for ATTO647N. However, direct excitation of unbound fluorescent seal DNAs (even under TIRF conditions) can lead to high background fluorescence and a lower signal to noise ratio (SNR). The use of fluorescence quenchers to reduce the background fluorescence was explored in the following experiments.

Unbound seal DNAs is single-stranded and forms random coils in solution, which could allow fluorescence quenchers at the ends of the seals to be in close proximity leading to contact-mediated fluorescence quenching [18]. Upon binding to a gapped-DNA, the seals no longer exist as random coils allowing the two ends of the seal to be apart, therefore, any contact induced fluorescence quenching would cease. In these experiments two different types of contact fluorescence quenchers were implemented, dark quenchers which do not fluoresce when excited and additional fluorophores.

Two 8 nt fully complementary seals were designed to test fluorescence quenching in solution: GSeq-8compl-T$^{ATTOS4TN P28, DABCYL P35}$ with the fluorescence quencher DABCYL at position 8 and GSeq-8compl-T$^{ATTO647N P28,P35}$ with an additional ATTO647N added at position 8.

When illuminated at 635nm (Fig. 8), the average SNR for GSeq-8compl-T$^{ATTO647N P28,P35}$ was very high for 30 nM, high for 100nM and moderate for 500 nM. The average SNR for GSeq-8compl-T$^{ATTO647N P28, DABCYL P35}$ was high for 30 nM, moderate for 50nM and no signal could be recorded for 500 nM. Both seals showed fluorescence quenching when in solution with GSeq-8compl-T$^{ATTO647N P28,P35}$ having a higher SNR for all of the concentrations due to the presence of a second fluorophore.

GSeq-8compl-T$^{ATTO647N P28,P35}$ and GSeq-8compl-T$^{ATTO647N P28, DABCYL P35}$ samples (at a concentration of 500 nM) were also illuminated with 532nm to observe transient binding through FRET. Binding events were observed for both seals (Fig. 9) proving that contact quenching was terminated when the seals bound to the gapped-DNA constructs.

GSeq-8compl-T$^{ATTO647N\ P28,P35}$ showed an intermediate FRET efficiency of 0.5 after E=0.9 for some binding events, (Fig. 9). A value of E=0.5 is expected for fluorophores of 16-20 bp separation. The ATTO647N, at position 8 on the seal was 16 bps from the Cy3B fluorophore on the gap DNA construct. Hence, this intermediate FRET efficiency can be attributed to FRET between the Cy3B at position 18 and ATTO647N at position 35, when the ATTO647N at position 28 could no longer participate in FRET.

Further fluorescence quenching experiments were conducted with triply labelled 7 nt seals. The seals showed fluorescence quenching in solution, however, no binding events were recorded. It is possible that the third fluorophore prevented binding or quenching continued when the seals bound. Triply labelled seals may still work, but additional optimization in terms of fluorophores, linkers and fluorophore separations will be necessary.

Both GSeq-8compl-T$^{ATTO647N\ P28,P35}$ and GSeq-8compl-T$^{ATTO647N\ P28,\ DABCYL\ P35}$ showed successful implementation of fluorescence quenching with no hindrance in binding for the seals. The success of fluorescence quenching with GSeq-8compl-T$^{ATTO647N\ P28,P35}$ allows a higher seal concentration necessary for high throughput, with previously working concentrations limited to 30nM - 100 nM.

3.1.5 A-T exclusive binding region

The previous gapped-DNA binding sequences had a majority of G-C pair, however, Gap-Seq needs to be applicable to all possible DNA sequences. Regions of exclusively A/T bases result in weaker hybridisation as strong bases form three hydrogen bonds as opposed to two hydrogen bonds for weak bases. To test Gap-Seq for regions of weak binding a 7 nt gapped-DNA construct was designed to have a binding sequence of only A or T bases, inspired by a common promoter region sequence 5'-TATAATA-3'.

First, 100nM GS7-ATrich-T$^{ATTO647N,P28,P34}$ was suspended in the standard imaging buffer with the surface immobilised 7 nt gapped-DNA with an A-T exclusive gap. Short transient binding events of $\leq 1$ second were observed (Fig. 10).

Tetramethylammonium chloride (TMAC) increases duplex stability for A-T rich regions by equalising the melting temperature between majority A-T regions and majority G-C sequences [19]. A second experiment was conducted with 3M TMAC present in the imaging buffer. The bound time of the seals increased to > 1 second in the presence of 3M TMAC (Fig. 10). The experiments conducted for the A-T exclusive binding region have shown Gap-Seq to be robust for all DNA sequences.

### 3.2 Proof of concept experiments for Steps 1 & 2

### 3.2.1 DNA Biosensor - CAP binding (Step 1)

As part of the development of the assay to link phenotype to DNA sequence, experiments were conducted to image the binding of catabolite activator protein (CAP) to surface immobilized dsDNA. CAP is a transcription factor that regulates the transcription of the lac Operon in *E. coli* by stabilising the binding of RNA polymerase (RNAP). CAP recognizes a two fold symmetric 22 bp consensus binding sequence 5'-AAATGTGATCTAGATCACATTT-3' [20] (SEQ ID NO: 1; Fig. 11b) and binds only in the presence of adenosine monophosphate (cAMP). When bound, CAP bends DNA by approximately 80 °, through primary and secondary kinks to stabilise binding [21] (Fig. 11a).

Exploiting CAP bending of DNA upon binding, Crawford et al. [22] designed a labeled biosensor DNA to detect CAP binding through a decrease in FRET efficiency from 0.5 to 0.25 (Fig. 12a).

Experiments were conducted with the biosensor DNA immobilised on the surface in the presence of CAP in CAP binding buffer. When illuminated at 532nm a FRET interconversion between an 0.4-0.5 state and a 0.1-0.2 state was recorded, signifying CAP binding (Fig. 12b).

The observation of single-molecule CAP binding has shown single-molecule protein-DNA interactions can be recorded with surface immobilised DNA, proving the viability of Step 1 of the assay to link phenotype to DNA/RNA sequence.

### 3.2.2 *In situ* denaturing and re-annealing of double stranded DNA (Step 2)

To establish creating a gapped-DNA construct from a dsDNA a protocol was developed to denature and re-anneal DNA. Using the DNA FRET standard (16 bp separation between fluorophores - Ev0.4-0.5) an experiment was conducted to denature and re-anneal dsDNA *in situ* by observing FRET efficiency changes (532nm illumination). First, the DNA FRET standards were imaged allowing doubly labelled DNA to be identified (N=370) and a majority E=0.45 recorded (Fig. 13a). After a 1 minute incubation with 10mM sodium hydroxide (NaOH) the same field of view was imaged again, with only 2 doubly labelled DNA FRET standards remaining (Fig. 13b). No distribution of E at 0.45 was recorded proving the success in denaturing the DNA FRET standard. The sample was then incubated with 50nM STD45-T$^{AT647N,P28}$ for 1 minute, washed and imaged. After illumination at 532nm 136 DNA FRET standards were observed (Fig. 13c) along with a distribution of E at 0.45. The reappearance of E=0.45 shows the success of re-annealing the DNA FRET standard. E distributions at 0.15 are due to experimental noise.

Not all DNA FRET standards re-annealed, with a re-annealing efficiency of 35% observed. Quantification is hindered by the photobleaching of the donor fluorophore attached to the immobilised bottom strand as photobleaching prevents detection of reannealed dsDNA.

The results show a working protocol was developed for in situ denaturing and reannealing of dsDNA by washing with 10mM NaOH and then reintroducing the complementary top strand. The technique will be developed further to anneal *in situ* the two top strands which flank the DNA gap to create gapped-DNA constructs.

**Further Results**

With reference to Figures 16 and 17 the data establishes the ability to detect single-molecule kinetic phenotypes from pools of single-molecules to which the DNA-binding protein CAP is binding (Figure 16), and show that there is a significant difference in the detected single-molecule phenotypes for dsDNAs that differ only by a single base (Figure 17).

With reference to Figure 18 the proof of concept is provided for the ability to collect a single-molecule kinetic phenotype from a single molecule, to create a gap on the same molecule, and to interrogate the sequence of the same molecule using transient hybridization information.

Figure 19 provides quantitative proof of concept that sequence information of a ssDNA in a DNA gap can be obtained through transient hybridization of DNA strands with different sequences (with the complementary seal 9-nt DNA binding significantly longer than the single-basepair mismatch DNA).

Figure 20 provides quantitative proof of concept that sequence information of a ssDNA in a DNA gap can be obtained through transient hybridization of DNA strands with different sequences including universal bases (with the complementary seal 13-nt DNA binding significantly longer than the single-basepair mismatch DNA).

Figure 21 provides quantitative proof of concept that sequence information of a ssDNA in a DNA gap can be obtained through transient hybridization of DNA strands with different sequences including degenerate bases (with the complementary seal 7-nt DNA binding significantly longer than the single-basepair mismatch DNA).

**4. Conclusion**

The experiments documented in this report have shown the development of an assay to link single-molecule phenotype and DNA sequence. The majority of the results focus on the development of Gap-Seq; a single-molecule DNA/RNA sequencing technique based on the transient binding of labelled oligos to gapped-DNA constructs.

First, a protocol was developed to successfully observe transient DNA binding of 12 nt and 9 nt labelled seals to a 15 nt gapped-DNA construct. For 9nt, 8nt and 6nt seals experiments were then able to detect a single bp mismatch between the seal and the gapped-DNA construct by analysis of the binding kinetics, showing a way to sequence a base. The robustness of Gap-Seq was proved by using a gapped-DNA binding region of exclusively A/T bases. Binding to this region was observed for a 7nt seal with 3M TMAC stabilising the binding. Contact fluorescence quenching of unbound seals was also successfully implemented by using the dark quencher DABCYL and a secondary fluorophore located at the 3' end of the seal. The addition of a secondary fluorophore increased the SNR of bound seals when directly excited, therefore, all further seals were designed with a secondary fluorophore.

Experiments were also conducted as proof of principle for Step 1 and Step 2 of the assay to link protein-DNA interaction to sequence. Single-molecule CAP-DNA binding events were recorded for surfaced immobilised DNA and a protocol was developed denature and re-anneal dsDNA *in situ.*

Overall, this report has documented a method to link single molecule phenotype to sequence, largely through the development of the novel single molecule sequencing technique Gap-Seq. In the future, Gap-Seq will be fully developed and implemented to study protein-DNA/RNA interactions.

**References**

[1] Watson, J. & Crick, F. A Structure for Deoxyribose Nucleic Acid. Nature 171(4356) 737{738 (1953).

[2] Sanger, F. & Nicklen, S. DNA sequencing with chain-terminating. Proceedings of the National Academy of Sciences of the United States of America 74(12) 5463{5467 (1977).

[3] Maxam, A. M. & Gilbert, W. A new method for sequencing DNA. Proceedings of the National Academy of Sciences of the United States of America 74(2) 560{564 (1977).

[4] Eid, J., Fehr, A., Gray, J., Luong, K., Lyle, J., Otto, G., Peluso, P. & Rank, D. Real-Time DNA Sequencing from Single Polymerase Molecules. Science 323(January) 133{139 (2009).

[5] Clarke, J.,Wu, H.-c., Jayasinghe, L., Patel, A., Reid, S. & Bayley, H. Continuous base identification for single-molecule nanopore DNA sequencing. Nature Nanotechnology 4(April) 265{270 (2009).

[6] Rhoads, A. & Au, K. F. PacBio Sequencing and Its Applications. Genomics Proteomics Bioinformatics 13 278{289 (2015).

[7] Oxford Nanopore Technologies. nanoporetech.com/how-it-works. Accessed 23.08.2017 .

[8] Drmanac, R., Brukner, I., Labat, I., Brukner, I. & Crkvenjakov, R. Sequencing of megabase plus DNA by hybridization : Theory of the Method. Genomics 7543 114{128 (1989).

[9] Bains, W. & Smith, G. A Novel Method for Nucleic Acid Sequence Determination. Journal of Theoretical Biology 135 303{307 (1988).

[10] Lysov, V., Floretiev, V., Khorlyn, A., Khrapko, K., Shick, V. & Mirzabekov, A. DNAsequencing by hybridisation with oligonucletides. Dokl. Acad. Sci. USSR 303 1508{1511 (1988).

[11] Jungmann, R., Steinhauer, C., Scheible, M., Kuzyk, A., Tinnefeld, P. & Simmel, F. C. Single-molecule kinetics and super-resolution microscopy by fluorescence imaging of transient binding on DNA origami. Nano Letters 10(11) 4756 {4761 (2010).

[12] Heilemann, M., Hwang, L. C., Lymperopoulos, K. & Kapanidis, A. N. Single-Molecule FRET Analysis of Protein DNA Complexes. In Methods in Molecular Biology, vol. 543, 503{521 (2009).

[13] Joo, C. & Ha, T. Single-Molecule FRET with Total Internal Reflection Microscopy. In Single-Molecule Techniques: A Laboratory Manual, 3{36 (2008).

[14] Kapanidis, A. N., Lee, N. K., Laurence, T. A., Doose, S., Margeat, E. & Weiss, S. Fluorescence-aided molecule sorting: analysis of structure and interactions by alternating-laser excitation of single molecules. Proceedings of the National Academy of Sciences of the United States of America 101(24) 8936{41 (2004).

[15] Kapanidis, A., Jing, B. & Crawford, R. Compact Microscope (2015).

[16] Dupuis, N. F., Holmstrom, E. D. & Nesbitt, D. J. Single-molecule kinetics reveal cation-promoted DNA duplex formation through ordering of single-stranded helices. Biophysical Journal 105(3) 756{766 (2013).

[17] Cisse, I. I., Kim, H. & Ha, T. A rule of seven in Watson-Crick base-pairing of mismatched sequences. Nature Structural & Molecular Biology 19(6) 623{627 (2012).

[18] Lakowicz, J. Principles of Fluorescence Spectroscopy. Third edit edn. Springer (2006).

[19] Melchior, W. B. & Hippel, P. Alteration of the Relative Stability of dA-dT and dG-dC Base Pairs in DNA. Proceedings of the National Academy of Sciences of the United States of America 70(2) 298{302 (1973).

[20] Ebright, R. H., Ebright, Y. W., Gunasekera, A. & Brunswick, N. Consensus DNA site for the Escherichia coli catabolite gene activator protein (CAP): CAP exhibits a 450-fold higher a_nity for the consensus DNA site than for the E.coli lac DNA site. Nucleic Acids Research 17(23) 16{18 (1989).

[21] Lawson, C. L., Swigon, D., Murakami, K. S., Darst, S. A., Helen, M. & Ebright, R. H. Catabolite activator protein (CAP): DNA binding and transcription activation. Current Opinion in Structural Biology 14(1) 10{20 (2004).

[22] Crawford, R., Kelly, D. J. & Kapanidis, A. N. A Protein Biosensor That Relies on Bending of Single DNA Molecules. Chemphyschem 13 918{922 (2012).

[23] Bauer, D. L. V., Plochowietz, A., Dulin, D., Malinen, A. M., Bakermans, J. J. W., Kaller, M. & Kapanidis, A. N. Sequence-Encoded Pause Signals Modulate Initial Transcription in Bacteria. Manuscript in Preparation (2017).

**Claims**

1. A method of sequencing a nucleic acid molecule, the method comprising:

   (a) providing the nucleic acid immobilised on a surface;
   (b) forming a single-stranded gap section by partially duplexing the nucleic acid such that the single-stranded gap section is flanked by duplex sections, wherein the sequence to be sequenced is a sequence of the single-stranded gap section;
   (c) providing a set of at least four fluorescently labelled oligonucleotide probes, wherein each fluorescently labelled oligonucleotide probe is up to 20 nucleotides in length, and equal in length to the single-stranded gap section, and each probe comprising:

      (i) a nucleotide X at a position arranged to oppose a nucleotide of the gap section of the immobilised nucleic acid, wherein such nucleotide is the interrogated nucleotide, and
      (ii) universal or degenerate bases arranged to oppose some or all of the remaining nucleotide positions of the gap section,

wherein the at least four fluorescently labelled oligonucleotide probes comprise:

- a first fluorescently labelled oligonucleotide probe wherein nucleotide X is a cytosine;
- a second fluorescently labelled oligonucleotide probe wherein nucleotide X is a guanine:
- a third fluorescently labelled oligonucleotide probe wherein nucleotide X is an adenine; and
- a fourth fluorescently labelled oligonucleotide probe wherein nucleotide X is a thymine or uracil;

(d) detecting binding, or absence thereof, of the fluorescently labelled oligonucleotide probes with the gap section of the immobilised nucleic acid, wherein the identity of the interrogated nucleotide of the gap section of the immobilised nucleic acid is identified as the complementary base of the nucleotide X of the fluorescently labelled oligonucleotide probe that has the highest incidence of binding;

(e) repeating steps (c) and (d) for interrogating subsequent nucleotide positions of the gap section of the immobilised nucleic acid until sufficient nucleotides of the gap section have been identified to be able to determine a sequence.

2. The method according to claim 1, wherein interrogating subsequent nucleotide positions of the gap section of the immobilised nucleic acid comprises providing a further set of at least four fluorescently labelled oligonucleotide probes, wherein the position of nucleotide X is changed to oppose a different/subsequent nucleotide to be interrogated of the immobilised nucleic acid; and/or

wherein the set of at least four fluorescently labelled oligonucleotide probes are provided together and a different fluorophore is used for each of the four different oligonucleotide probes; and/or

wherein detecting the binding, or absence thereof, of the fluorescently labelled oligonucleotide probes with the gap section of the immobilised nucleic acid comprises detecting the transient binding, or absence thereof, of the fluorescently labelled oligonucleotide probes with the gap section of the immobilised nucleic acid.

3. The method according to any preceding claim, wherein partially duplexing the nucleic acid comprises hybridising a pair of gap-flanking nucleic acid strands that are each complementary to a different section of the nucleic acid to be duplexed, wherein the single-stranded gap section is formed therebetween; or

wherein the partially duplexed nucleic acid comprising a single stranded gap-section is formed by circularising the nucleic acid with a splint nucleic acid that is arranged to hybridise to each end of the nucleic acid; and/or

wherein the sequences of the nucleic acid to be duplexed are designed or built into the nucleic acid, such that they flank a sequence of unknown/random nucleotides to be interrogated.

4. The method according to any preceding claim, wherein the fluorescently labelled oligonucleotide probes comprise universal bases arranged to oppose some or all of the remaining nucleotide positions of the gap section; and/or wherein the fluorescently labelled oligonucleotide probes comprise degenerate bases arranged to oppose some or all of the remaining nucleotide positions of the gap section.

5. The method according to any preceding claim, wherein the binding of a fluorescently labeled oligonucleotide probe to the gap-section of the nucleic acid is detected by measurement of Förster resonance energy transfer (FRET) between the fluorophore on the fluorescently labeled oligonucleotide probe and a second fluorophore;

optionally wherein the second fluorophore is tagged on the immobilized nucleic acid molecule or duplex thereof; or

wherein the second fluorophore is tagged as a second fluorophore on the fluorescently labeled oligonucleotide probe.

6. The method according to any preceding claim, wherein the nucleic acid comprises a combination of pre-determined sequence and unknown/randomised sequence; and/or

wherein the nucleic acid is provided in the form of a library of nucleic acids comprising a randomised nucleotide region; and/or

wherein the nucleic acid comprises a functionality that allows its surface-immobilisation on a surface; and/or wherein the immobilised nucleic acids are spatially arranged on a glass surface; or

wherein the immobilised nucleic acid is immobilised *in situ* in a cell and the cell is chemically fixed or immobilised by capture in a microfluidic channel.

7. The method according to any preceding claim, wherein the method further provides the step of amplifying the immobilised nucleic acid;
optionally wherein the amplification comprises a rolling circle amplification (RCA) of the immobilised nucleic acid.

8. A method of single-molecule phenotyping and sequencing of a nucleic acid molecule, the method comprising:

a) providing the nucleic acid molecule immobilised to a surface;
b) testing for a phenotype of the immobilised nucleic acid molecule; and
c) determining the sequence of the immobilised nucleic acid molecule such that an observed phenotype is linked to the sequence, wherein the nucleic acid is sequenced by the method of sequencing according to any of claims 1-7;
optionally wherein the testing for a phenotype of the immobilised nucleic acid molecule comprises testing for binding of the nucleic acid to a target molecule;
further optionally wherein the target molecule comprises a small molecule, an oligopeptide, a polypeptide, a nucleic acid, or a lipid molecule.

9. The method according to claim 8, wherein the phenotypical analysis is on the double-stranded form of the nucleic acid, where the nucleic acid is provided double-stranded; and/or
wherein following the phenotypic analysis, the method further comprises a denaturing and/or wash step to remove any interacting or non-interacting molecules or denaturing the nucleic acid, prior to sequencing the nucleic acid.

10. A method of single-molecule phenotyping and identification of a molecule, wherein the molecule is tagged with a nucleic acid and identified by sequencing of the tagged nucleic acid, the method comprising:

a) providing the molecule immobilised to a surface;
b) testing for a phenotype of the immobilised molecule; and
c) determining the sequence of the tagged nucleic acid molecule such that an observed phenotype is linked to the tagged nucleic acid sequence, wherein the tagged nucleic acid is sequenced by the method of sequencing according to any of claims 1-7; optionally wherein the immobilised molecule comprises a protein that is fused to a nucleic acid.

11. A method of single-cell phenotyping and sequencing of a nucleic acid molecule associated with the phenotype of the cell, the method comprising:

a) providing a population of cells comprising modifications in a nucleic acid sequence of interest;
b) observing a phenotype of individual cells, wherein the cells are fixed to a surface before, during or after the phenotypic observation, such that the nucleic acid sequence is also immobilised;
c) sequencing the nucleic acid sequence by the method of sequencing according to claims 1-7, such that an observed phenotype of the cell is linked to the nucleic acid sequence; optionally wherein the population of cells comprise nucleic acid sequence from a randomised library of nucleic acid modifications.

12. The method according to claim 10 or 11, wherein the nucleic acid sequence comprises at least part of a gene sequence that is modified.

**Patentansprüche**

1. Verfahren zum Sequenzieren eines Nukleinsäuremoleküls, wobei das Verfahren Folgendes umfasst:

(a) Bereitstellen der Nukleinsäure, immobilisiert auf einer Oberfläche;
(b) Bilden eines einzelsträngigen Lückenabschnitts durch derartiges teilweises Duplexen der Nukleinsäure, dass der einzelsträngige Lückenabschnitt von Duplexabschnitten flankiert wird, wobei die zu sequenzierende Sequenz eine Sequenz des einzelsträngigen Lückenabschnitts ist;
(c) Bereitstellen eines Satzes von mindestens vier fluoreszent markierten Oligonukleotidsonden, wobei jede fluoreszent markierte Oligonukleotidsonde eine Länge von bis zu 20 Nukleotiden und eine gleiche Länge wie der einzelsträngige Lückenabschnitt aufweist und jede Sonde Folgendes umfasst:

(i) ein Nukleotid X an einer Position, die dazu angeordnet ist, einem Nukleotid des Lückenabschnitts der immobilisierten Nukleinsäure gegenüberzuliegen, wobei ein solches Nukleotid das abgefragte Nukleotid ist, und
(ii) universale oder degenerierte Basen, die dazu angeordnet sind, einigen oder allen der verbleibenden Nukleotidpositionen des Lückenabschnitts gegenüberzuliegen,

wobei die mindestens vier fluoreszent markierten Oligonukleotidsonden Folgendes umfassen:

- eine erste fluoreszent markierte Oligonukleotidsonde, wobei das Nukleotid X ein Cytosin ist;
- eine zweite fluoreszent markierte Oligonukleotidsonde, wobei das Nukleotid X ein Guanin ist;
- eine dritte fluoreszent markierte Oligonukleotidsonde, wobei das Nukleotid X ein Adenin ist; und
- eine vierte fluoreszent markierte Oligonukleotidsonde, wobei das Nukleotid X ein Thymin oder ein Uracil ist;

(d) Detektieren einer Bindung oder einer Abwesenheit davon der fluoreszent markierten Oligonukleotidsonden an den Lückenabschnitt der immobilisierten Nukleinsäure, wobei die Identität des abgefragten Nukleotids des Lückenabschnitts der immobilisierten Nukleinsäure als die komplementäre Base des Nukleotids X der fluoreszent markierten Oligonukleotidsonde identifiziert wird, die das höchste Vorkommen der Bindung aufweist;
(e) Wiederholen der Schritte (c) und (d) zum Abfragen nachfolgender Nukleotidpositionen des Lückenabschnitts der immobilisierten Nukleinsäure, bis genug Nukleotide des Lückenabschnitts identifiziert sind, um eine Sequenz bestimmen zu können.

2. Verfahren nach Anspruch 1, wobei das Abfragen nachfolgender Nukleotidpositionen des Lückenabschnitts der immobilisierten Nukleinsäure Bereitstellen eines weiteren Satzes von mindestens vier fluoreszent markierten Oligonukleotidsonden umfasst, wobei die Position des Nukleotids X geändert wird, um einem unterschiedlichen/-nachfolgenden abzufragenden Nukleotid der immobilisierten Nukleinsäure gegenüberzuliegen; und/oder

wobei der Satz von mindestens vier fluoreszent markierten Oligonukleotidsonden sämtlich bereitgestellt wird und ein unterschiedliches Fluorophor für jede der vier unterschiedlichen Oligonukleotidsonden verwendet wird; und/oder
wobei das Detektieren der Bindung oder der Abwesenheit davon der fluoreszent markierten Oligonukleotidsonden an den Lückenabschnitt der immobilisierten Nukleinsäure Detektieren der vorübergehenden Bindung oder der Abwesenheit davon der fluoreszent markierten Oligonukleotidsonden an den Lückenabschnitt der immobilisierten Nukleinsäure umfasst.

3. Verfahren nach einem der vorhergehenden Ansprüche, wobei das teilweise Duplexen der Nukleinsäure Hybridisieren eines Paares lückenflankierender Nukleinsäurestränge umfasst, die jeweils komplementär zu einem unterschiedlichen Abschnitt der zu duplexenden Nukleinsäure sind, wobei der einzelsträngige Lückenabschnitt dazwischen gebildet wird; oder

wobei die teilweise geduplexte Nukleinsäure, umfassend einen einzelsträngigen Lückenabschnitt, durch Zirkularisieren der Nukleinsäure mit einer Splint-Nukleinsäure gebildet wird, die dazu angeordnet ist, jedes Ende der Nukleinsäure zu hybridisieren; und/oder
wobei die Sequenzen der zu duplexenden Nukleinsäure derart entworfen oder in die Nukleinsäure eingebaut sind, dass sie eine Sequenz abzufragender unbekannter/zufälliger Nukleotide flankieren.

4. Verfahren nach einem der vorhergehenden Ansprüche, wobei die fluoreszent markierten Oligonukleotidsonden universale Basen umfassen, die dazu angeordnet sind, einigen oder allen der verbleibenden Nukleotidpositionen des Lückenabschnitts gegenüberzuliegen; und/oder
wobei die fluoreszent markierten Oligonukleotidsonden degenerierte Basen umfassen, die dazu angeordnet sind, einigen oder allen der verbleibenden Nukleotidpositionen des Lückenabschnitts gegenüberzuliegen.

5. Verfahren nach einem der vorhergehenden Ansprüche, wobei die Bindung einer fluoreszent markierten Oligonukleotidsonde an den Lückenabschnitt der Nukleinsäure durch eine Messung eines Förster-Resonanzenergietransfers (FRET) zwischen dem Fluorophor auf der fluoreszent markierten Oligonukleotidsonde und einem zweiten Fluorophor detektiert wird;

gegebenenfalls wobei das zweite Fluorophor auf dem immobilisierten Nukleinsäuremolekül oder dem Duplex davon getaggt wird; oder

wobei das zweite Fluorophor als ein zweites Fluorophor auf der fluoreszent markierten Oligonukleotidsonde getaggt wird.

6. Verfahren nach einem der vorhergehenden Ansprüche, wobei die Nukleinsäure eine Kombination einer vorbestimmten Sequenz und einer unbekannten/randomisierten Sequenz umfasst; und/oder

wobei die Nukleinsäure in der Form einer Bibliothek von Nukleinsäuren, umfassend eine randomisierte Nukleotidregion, bereitgestellt wird; und/oder
wobei die Nukleinsäure eine Fähigkeit umfasst, die ihre Oberflächenimmobilisierung auf einer Oberfläche ermöglicht; und/oder
wobei die immobilisierten Nukleinsäuren auf einer Glasoberfläche räumlich angeordnet werden; oder
wobei die immobilisierte Nukleinsäure *in situ* in einer Zelle immobilisiert wird und die Zelle durch Erfassung in einem mikrofluidischen Kanal chemisch fixiert oder immobilisiert wird.

7. Verfahren nach einem der vorhergehenden Ansprüche, wobei das Verfahren ferner den Schritt von Amplifizieren der immobilisierten Nukleinsäure bereitstellt;
gegebenenfalls wobei die Amplifikation eine Rolling-Circle-Amplifikation (RCA) der immobilisierten Nukleinsäure umfasst.

8. Verfahren zum Einzelmolekülphänotypisieren und Sequenzieren eines Nukleinsäuremoleküls, wobei das Verfahren Folgendes umfasst:

a) Bereitstellen des Nukleinsäuremoleküls, immobilisiert an einer Oberfläche;
b) Prüfen auf einen Phänotypus des immobilisierten Nukleinsäuremoleküls; und
c) derartiges Bestimmen der Sequenz des immobilisierten Nukleinsäuremoleküls, dass ein beobachteter Phänotypus mit der Sequenz verknüpft wird, wobei die Nukleinsäure durch das Verfahren zum Sequenzieren nach einem der Ansprüche 1-7 sequenziert wird;
gegebenenfalls wobei das Prüfen auf einen Phänotypus des immobilisierten Nukleinsäuremoleküls Prüfen auf eine Bindung der Nukleinsäure an ein Zielmolekül umfasst;
ferner gegebenenfalls wobei das Zielmolekül ein kleines Molekül, ein Oligopeptid, ein Polypeptid, eine Nukleinsäure oder ein Lipidmolekül umfasst.

9. Verfahren nach Anspruch 8, wobei die phänotypische Analyse an der doppelsträngigen Form der Nukleinsäure stattfindet, wo die Nukleinsäure doppelsträngig bereitgestellt wird; und/oder
wobei das Verfahren folgend auf die phänotypische Analyse ferner einen Denaturierungs- und/oder Waschschritt, um etwaige interagierende oder nicht interagierende Moleküle zu entfernen, oder Denaturieren der Nukleinsäure im Vorfeld des Sequenzierens der Nukleinsäure umfasst.

10. Verfahren zum Einzelmolekülphänotypisieren und Identifizieren eines Moleküls, wobei das Molekül mit einer Nukleinsäure getaggt und durch Sequenzieren der getaggten Nukleinsäure identifiziert wird, wobei das Verfahren Folgendes umfasst:

a) Bereitstellen des Moleküls, immobilisiert an einer Oberfläche;
b) Prüfen auf einen Phänotypus des immobilisierten Moleküls; und
c) derartiges Bestimmen der Sequenz des getaggten Nukleinsäuremoleküls, dass ein beobachteter Phänotypus mit der getaggten Nukleinsäuresequenz verknüpft wird, wobei die getaggte Nukleinsäure durch das Verfahren zum Sequenzieren nach einem der Ansprüche 1-7 sequenziert wird; gegebenenfalls wobei das immobilisierte Molekül ein Protein umfasst, das mit einer Nukleinsäure fusioniert wird.

11. Verfahren zum Einzelzellphänotypisieren und Sequenzieren eines Nukleinsäuremoleküls, das dem Phänotypus der Zelle zugeordnet ist, wobei das Verfahren Folgendes umfasst:

a) Bereitstellen einer Zellpopulation, umfassend Modifikationen in einer Nukleinsäuresequenz von Interesse;
b) Beobachten eines Phänotypus einzelner Zellen, wobei die Zellen vor, während oder nach der phänotypischen Beobachtung derart an einer Oberfläche fixiert werden, dass die Nukleinsäuresequenz ebenfalls immobilisiert wird;

c) derartiges Sequenzieren der Nukleinsäuresequenz durch das Verfahren zum Sequenzieren nach Anspruch 1-7, dass ein beobachteter Phänotypus der Zelle mit der Nukleinsäuresequenz verknüpft wird, gegebenenfalls wobei die Zellpopulation eine Nukleinsäuresequenz aus einer randomisierten Bibliothek von Nukleinsäuremodifikationen umfasst.

12. Verfahren nach Anspruch 10 oder 11, wobei die Nukleinsäuresequenz mindestens einen Teil einer Gensequenz umfasst, die modifiziert wird.

**Revendications**

1. Procédé de séquençage d'une molécule d'acide nucléique, le procédé comprenant :

(a) la fourniture de l'acide nucléique immobilisé sur une surface ;
(b) la formation d'une section d'espacement simple brin en duplexant partiellement l'acide nucléique de telle sorte que la section d'espacement simple brin soit flanquée par des sections duplex, dans lequel la séquence à séquencer est une séquence de la section d'espacement simple brin ;
(c) la fourniture d'un ensemble d'au moins quatre sondes oligonucléotidiques marquées par fluorescence, dans lequel chaque sonde oligonucléotidique marquée par fluorescence mesure jusqu'à 20 nucléotides de longueur et est égale en longueur à la section d'espacement simple brin, et chaque sonde comprenant :

(i) un nucléotide X à une position agencée de manière à s'opposer à un nucléotide de la section d'espacement de l'acide nucléique immobilisé, dans lequel ledit nucléotide est le nucléotide interrogé, et
(ii) des bases universelles ou dégénérées agencées de manière à s'opposer à certaines ou à toutes les positions nucléotidiques restantes de la section d'espacement,

dans lequel les au moins quatre sondes oligonucléotidiques marquées par fluorescence comprennent :

- une première sonde oligonucléotidique marquée par fluorescence dans lequel le nucléotide X est une cytosine ;
- une seconde sonde oligonucléotidique marquée par fluorescence dans lequel le nucléotide X est une guanine ;
- une troisième sonde oligonucléotidique marquée par fluorescence dans lequel le nucléotide X est une adénine ; et
- une quatrième sonde oligonucléotidique marquée par fluorescence dans lequel le nucléotide X est une thymine ou un uracile ;

(d) la détection de liaison, ou l'absence de liaison, des sondes oligonucléotidiques marquées par fluorescence avec la section d'espacement de l'acide nucléique immobilisé, l'identité du nucléotide interrogé de la section d'espacement de l'acide nucléique immobilisé étant identifiée comme la base complémentaire du nucléotide X de la sonde oligonucléotidique marquée par fluorescence qui présente l'incidence de liaison la plus élevée ;
(e) la répétition des étapes (c) et (d) pour interroger les positions nucléotidiques ultérieures de la section d'espacement de l'acide nucléique immobilisé jusqu'à ce que suffisamment de nucléotides de la section d'espacement aient été identifiés pour pouvoir déterminer une séquence.

2. Procédé selon la revendication 1, dans lequel l'interrogation des positions nucléotidiques ultérieures de la section d'espacement de l'acide nucléique immobilisé comprend la fourniture d'un autre ensemble d'au moins quatre sondes oligonucléotidiques marquées par fluorescence, la position du nucléotide X étant modifiée pour s'opposer à un nucléotide différent/ultérieur à interroger de l'acide nucléique immobilisé ; et/ou

dans lequel l'ensemble d'au moins quatre sondes oligonucléotidiques marquées par fluorescence sont fournis ensemble et un fluorophore différent est utilisé pour chacune des quatre sondes oligonucléotidiques différentes ; et/ou
dans lequel la détection de liaison, ou de l'absence de celle-ci, des sondes oligonucléotidiques marquées par fluorescence avec la section d'espacement de l'acide nucléique immobilisé comprend la détection de la liaison transitoire, ou de l'absence de celle-ci, des sondes oligonucléotidiques marquées par fluorescence avec la section d'espacement de l'acide nucléique immobilisé.

3. Procédé selon l'une quelconque des revendications précédentes, dans lequel le duplexage partiel de l'acide nucléique comprend l'hybridation d'une paire de brins d'acide nucléique flanquant un espacement, chacun étant complémentaire d'une section différente de l'acide nucléique à duplexer, la section d'espacement simple brin étant formée entre eux ; ou

   dans lequel l'acide nucléique partiellement duplexé comprenant une section d'espacement simple brin est formé par circularisation de l'acide nucléique avec un acide nucléique attelle qui est conçu pour s'hybrider à chaque extrémité de l'acide nucléique ; et/ou
   dans lequel les séquences de l'acide nucléique à duplexer sont conçues ou intégrées dans l'acide nucléique, de telle sorte qu'elles flanquent une séquence de nucléotides inconnus/aléatoires à interroger.

4. Procédé selon l'une quelconque des revendications précédentes, dans lequel les sondes oligonucléotidiques marquées par fluorescence comprennent des bases universelles agencées de manière à s'opposer à certaines ou à toutes les positions nucléotidiques restantes de la section d'espacement ; et/ou
   dans lequel les sondes oligonucléotidiques marquées par fluorescence comprennent des bases dégénérées agencées pour s'opposer à certaines ou à toutes les positions nucléotidiques restantes de la section d'espacement.

5. Procédé selon l'une quelconque des revendications précédentes, dans lequel la liaison d'une sonde oligonucléotidique marquée par fluorescence à la section d'espacement de l'acide nucléique est détectée par mesure du transfert d'énergie par résonance de Förster (FRET) entre le fluorophore sur la sonde oligonucléotidique marquée par fluorescence et un second fluorophore ;

   éventuellement dans lequel le second fluorophore est marqué sur la molécule d'acide nucléique immobilisée ou un duplex de celle-ci ; ou
   dans lequel le second fluorophore est marqué comme un second fluorophore sur la sonde oligonucléotidique marquée par fluorescence.

6. Procédé selon l'une quelconque des revendications précédentes, dans lequel l'acide nucléique comprend une combinaison d'une séquence prédéterminée et d'une séquence inconnue/randomisée ; et/ou

   dans lequel l'acide nucléique est fourni sous la forme d'une bibliothèque d'acides nucléiques comprenant une région nucléotidique randomisée ; et/ou
   dans lequel l'acide nucléique comprend une fonctionnalité qui permet son immobilisation en surface sur une surface ; et/ou
   dans lequel les acides nucléiques immobilisés sont disposés spatialement sur une surface de verre ; ou
   dans lequel l'acide nucléique immobilisé est immobilisé *in situ* dans une cellule et la cellule est fixée chimiquement ou immobilisée par capture dans un canal microfluidique.

7. Procédé selon l'une quelconque des revendications précédentes, dans lequel le procédé comprend en outre l'étape d'amplification de l'acide nucléique immobilisé ;
   éventuellement dans lequel l'amplification comprend une amplification en cercle roulant (RCA) de l'acide nucléique immobilisé.

8. Procédé de phénotypage et de séquençage d'une molécule d'acide nucléique unique, le procédé comprenant :

   a) la fourniture de la molécule d'acide nucléique immobilisée sur une surface ;
   b) le test d'un phénotype de la molécule d'acide nucléique immobilisée ; et
   c) la détermination de la séquence de la molécule d'acide nucléique immobilisée de telle sorte qu'un phénotype observé soit lié à la séquence, l'acide nucléique étant séquencé par le procédé de séquençage selon l'une quelconque des revendications 1 à 7 ;
   éventuellement dans lequel le test d'un phénotype de la molécule d'acide nucléique immobilisée comprend le test de liaison de l'acide nucléique à une molécule cible ;
   en outre, éventuellement, dans lequel la molécule cible comprend une petite molécule, un oligopeptide, un polypeptide, un acide nucléique ou une molécule lipidique.

9. Procédé selon la revendication 8, dans lequel l'analyse phénotypique porte sur la forme double brin de l'acide nucléique, où l'acide nucléique est fourni double brin ; et/ou
   dans lequel, suite à l'analyse phénotypique, le procédé comprend en outre une étape de dénaturation et/ou de lavage pour éliminer toutes les molécules en interaction ou non ou la dénaturation de l'acide nucléique, avant le séquençage de l'acide nucléique.

10. Procédé de phénotypage et d'identification d'une molécule unique, dans lequel la molécule est marquée avec un acide nucléique et identifiée par séquençage de l'acide nucléique marqué, le procédé comprenant :

a) la fourniture de la molécule immobilisée sur une surface ;
b) le test d'un phénotype de la molécule immobilisée ; et
c) la détermination de la séquence de la molécule d'acide nucléique marquée de telle sorte qu'un phénotype observé soit lié à la séquence d'acide nucléique marquée, l'acide nucléique marqué étant séquencé par le procédé de séquençage selon l'une quelconque des revendications 1 à 7 ; éventuellement dans lequel la molécule immobilisée comprend une protéine qui est fusionnée à un acide nucléique.

11. Procédé de phénotypage et de séquençage de cellule unique d'une molécule d'acide nucléique associée au phénotype de la cellule, le procédé comprenant :

a) la fourniture d'une population de cellules comprenant des modifications dans une séquence d'acide nucléique d'intérêt ;
b) l'observation d'un phénotype de cellules individuelles, dans lequel les cellules sont fixées à une surface avant, pendant ou après l'observation phénotypique, de telle sorte que la séquence d'acide nucléique est également immobilisée ;
c) le séquençage de la séquence d'acide nucléique par le procédé de séquençage selon les revendications 1 à 7, de telle sorte qu'un phénotype observé de la cellule soit lié à la séquence d'acide nucléique ; éventuellement dans lequel la population de cellules comprend une séquence d'acide nucléique provenant d'une bibliothèque randomisée de modifications d'acide nucléique.

12. Procédé selon la revendication 10 ou 11, dans lequel la séquence d'acide nucléique comprend au moins une partie d'une séquence génétique qui est modifiée.

# Figure 1

DNA/RNA library of $4^N$ sequences $\longrightarrow$ "molecular phenotype" for each DNA of the library

| | | | |
|---|---|---|---|
| immobilise library | functional **assay** (binding, reactions) | analyse kinetics of fluorescence change | "molecular phenotype" (rate constants, affinities structures) |
| A | B | C | D |

EP 3 682 033 B1

Figure 2

**Preparation for sequencing**
remove protein
remove top strand
add 2 short DNAs

**D: degenerate base or universal base**

A — prepare gapped DNA

B — readout strands ("seals") for $N_1$ transiently bind DNA

C — complementary seal binds more stably

D — call base $N_1$

$N_1 = A$

$N_1 = G$

$N_1 = C$

(Blue) TDDDD / ATTTT

(Red) CDDDD / GTTTT

(Green) GDDDD / CATTT

mol#1
mol#2
mol#3

## Figure 3

| A | B | C | D |
|---|---|---|---|
| remove $N_1$ seals, add $N_2$ seals | $N_2$ seals transiently bind DNA | complementary seal binds more stably | call base $N_2$ |

EP 3 682 033 B1

Figure 4

Figure 4 continued

Figure 5

# Figure 6

Figure 7

a) GS6-compl-T$^{AT647N,P28}$

b) GS6-mis3G-T$^{AT647N,P28}$

Figure 8

a) GS8-compl- T$^{AT647N,P28,P35}$

b) GS8-compl- T$^{AT647N,P28, DABCYL,P35}$

30 nM          100 nM          500 nM

Figure 9

Figure 10

# Figure 11

a)

b)

Figure 12

Figure 13

EP 3 682 033 B1

| Construct Name | Name of Strand | DNA sequence |
|---|---|---|
| Biotinylated Hairpin (15 nt gap) | Biotinylated Hairpin | 5' CTG CTG CCT CAG GCT CAT --A AGA GCC TGA GGC AGC AGA TTA TCG ACG GTG GAT GTA TGG TAA TGG GAC GAA GAA TGA GG-bio 3' (SEQ ID NO: 2) |
| | GSeq(1-27)-T$^{Cy3B,P18}$ | 5' CCT CAT TCT TCG TCC CAT TAC CAT ACA 3' (SEQ ID NO: 3) |
| 9 nt gap | GSeq(1-65)-B$^{Bio,P1}$ | 5' CAG TCA AGA GCC TGA GGC AGC AGA TTA TCG ACG GTG GAT GTA TGG TAA TGG GAC GAA GAA TGA GG-bio 3' (SEQ ID NO: 4) |
| | GSeq(1-27)-T$^{Cy3B,P18}$ | 5' CCT CAT TCT TCG TCC CAT TAC CAT ACA 3' (SEQ ID NO: 3) |
| | GSeq(37-65)-T | 5' GAT AAT CTG CTG CCT CAG GCT CTT GAC TG 3' (SEQ ID NO: 5) |
| 8 nt gap | GSeq(1-65)-B$^{Bio,P1}$ | 5' CAG TCA AGA GCC TGA GGC AGC AGA TTA TCG ACG GTG GAT GTA TGG TAA TGG GAC GAA GAA TGA GG-bio 3' (SEQ ID NO: 4) |
| | GSeq(1-27)-T$^{Cy3B,P18}$ | 5' CCT CAT TCT TCG TCC CAT TAC CAT ACA 3' (SEQ ID NO: 3) |
| | GSeq(36-65)-T | 5' CGA TAA TCT GCT GCC TCA GGC TCT TGA CTG 3' (SEQ ID NO: 6) |
| 6 nt gap | GSeq(1-65)-B$^{Bio,P1}$ | 5' CAG TCA AGA GCC TGA GGC AGC AGA TTA TCG ACG GTG GAT GTA TGG TAA TGG GAC GAA GAA TGA GG-bio 3' (SEQ ID NO: 4) |
| | GSeq(1-27)-T$^{Cy3B,P18}$ | 5' CCT CAT TCT TCG TCC CAT TAC CAT ACA 3' (SEQ ID NO: 3) |
| | GSeq(34-65)-T | 5' GTC GAT AAT CTG CTG CCT CAG GCT CTT GAC TG 3' (SEQ ID NO: 7) |
| 7 nt gap (A at P31) | GSeq(1-65)-A$^{P31}$-B$^{Bio,P1}$ | 5' CAG TCA AGA GCC TGA GGC AGC AGA TTA TCG ACG GAG GAT GTA TGG TAA TGG GAC GAA GAA TGA GG-bio 3' (SEQ ID NO: 8) |
| | GSeq(1-27)-T$^{Cy3B,P18}$ | 5' CCT CAT TCT TCG TCC CAT TAC CAT ACA 3' (SEQ ID NO: 3) |
| | GSeq(35-65)-T | 5' TCG ATA ATC TGC TGC CTC AGG CTC TTG ACT G 3' (SEQ ID NO: 9) |
| 7 nt gap (A-T exclusive) | GSeq-Atrich(1-65)-B$^{Bio,P1}$ | 5' CAG TCA AGA GCC TGA GGC AGC AGA TTA TCG AAT AAT ATT GTA TGG TAA TGG GAC GAA GAA TGA GG -bio 3' (SEQ ID NO: 10) |
| | GSeq(1-27)-T$^{Cy3B,P18}$ | 5' CCT CAT TCT TCG TCC CAT TAC CAT ACA 3' (SEQ ID NO: 3) |
| | GSeq(35-65)-T | 5' TCG ATA ATC TGC TGC CTC AGG CTC TTG ACT G 3' (SEQ ID NO: 9) |
| 13 nt gap | GSeq(1-65)-A$^{P31}$-B$^{Bio,P1}$ | 5' CAG TCA AGA GCC TGA GGC AGC AGA TTA TCG ACG GAG GAT GTA TGG TAA TGG GAC GAA GAA TGA GG-bio 3' (SEQ ID NO: 8) |
| | GSeq(1-27)-T$^{Cy3B,P18}$ | 5' CCT CAT TCT TCG TCC CAT TAC CAT ACA 3' (SEQ ID NO: 3) |
| | GSeq(41-65)-T | 5' ATC TGC TGC CTC AGG CTC TTG ACT G 3' (SEQ ID NO: 11) |

Figure 14

EP 3 682 033 B1

Figure 15

| | Sequence Name | DNA Sequence |
|---|---|---|
| Labelled Oligonucleotide Seals | GS12-compl-T[AT647N,P28] | 5' TCCACCGTCGAT 3' (SEQ ID NO: 12) |
| | GS9-compl-T[AT647N,P28] | 5' TCCACCGTC 3' |
| | GS6-compl-T[AT647N,P28] | 5' TCCACC 3' |
| | GS9-mis5G-T[ATTO647N,P28] | 5' TCC AGC GTC 3' |
| | GS9-mis3G-T[ATTO647N,P28] | 5' TCG ACC GTC 3' |
| | GS8-compl-T[AT647N,P28] | 5' TCC ACC GT 3' |
| | GS8-mis5G-T[ATTO647N,P28] | 5' TCC AGC GT 3' |
| | GS8-mis3G-T[ATTO647N,P28] | 5' TCG ACC GT 3' |
| | GS8-compl-T[AT647N,P28,CARBOX,P35] | 5' TCC ACC GT 3' |
| | GS8-compl-T[AT647N,P28,P35] | 5' TCC ACC GT 3' |
| | GS7-ATrich-T[AT647N,P28,P34] | 5' ATA TTA T 3' |
| | GS6-mis3G-T[AT647N,P28,P35] | 5' TCG ACC 3' |
| | GS13-N[10,11,12,13]-T[AT647N,P28,P35] | 5' TCC ACC GTC NNN N -3' (SEQ ID NO: 13) |
| | GS13-mis9G-N[10,11,12,13]-T[AT647N,P28,P35] | 5' TCC ACC GTG NNN N -3' (SEQ ID NO: 14) |
| | GS7-degen[P30,P32]-T[ATTO647N,P28,P34] | 5' TCN ANC G-3' |
| | GS7-mis4T-degen[P30,P32]-T[AT647N,P28,P34] | 5' TCN TNC G-3' |
| DNA FRET Standard | STD45-T[Bio,P1,Cy3,P45] | 5' TAA ATC TAA AGT AAC ATA AGG TAA CAT AAC GTA AGC TCA TTC GCG bio 3' (SEQ ID NO: 15) |
| | STD45-B[AT647N,P28] | 5' CGC GAA TGA GCT TAC GTT ATG TTA CCT TAT GTT ACT TTA GAT TTA 3' (SEQ ID NO: 16) |
| Biosensor DNA | Biosensor-T[Cy3B,P1,Bio,P78] | 5' CCC ACT GCC GAA TGT GAG TTA GCT CAC TCA CGA AAA ACC ACT GTC GAA AAA GCT CTA CGG GCT CTG GCG TCG G – bio 3' (SEQ ID NO: 17) |
| | Biosensor-B[AT647N,P78] | 5' GCG ACG CCA GAG CCC GTA GAG CCG ACA GTG GCG TGA GTG AGC TAA CTC ACA TTC GAA AAA GCA GTG GG 3' (SEQ ID NO: 18) |

Consensus CAP DNA

Figure 16

Weakened Consensus CAP DNA

Figure 16 continued

## Figure 17A

__Consensus CAP DNA__

| Model | ExpDec2 |
|---|---|
| Equation | y=A1*(exp(-x/t1)+A2*(exp(-x/t2)+y0 |
| A1 | 77.5 % |
| t1 | 1.00 ± 0.05 secs |
| A2 | 22.5 % |
| **t2** | **8.75 ± 0.32 secs** |

| Model | ExpDec2 |
|---|---|
| Equation | y=A1*(exp(-x/t1)+A2*(exp(-x/t2)+y0 |
| A1 | 82.3 % |
| t1 | 3.70 ± 1.14 secs |
| A2 | 17.7 % |
| **t2** | **87.2 ± 5.87 secs** |

## Figure 17B

__Weakened Consensus CAP DNA__

| Model | ExpDec2 |
|---|---|
| Equation | y=A1*(exp(-x/t1)+A2*(exp(-x/t2)+y0 |
| A1 | 78.7 % |
| t1 | 1.43 ± 0.06 secs |
| A2 | 21.3 % |
| **t2** | **6.75 ± 0.37 secs** |

| Model | ExpDec2 |
|---|---|
| Equation | y=A1*(exp(-x/t1)+A2*(exp(-x/t2)+y0 |
| A1 | 74.3 % |
| t1 | 4.40 ± 1.73 secs |
| A2 | 25.7 % |
| **t2** | **103.51 ± 8.54 secs** |

# Figure 18

## Figure 19

# Figure 20

GS13-N$^{10,11,12,13}$-$_T$AT647N,P28,P35

GS13-mis9G-N$^{10,11,12,13}$-$_T$AT647N,P28,P35

| Model | ExpDec2 |
|---|---|
| Equation | y=A1*(exp(-x/t1)+A2*(exp(-x/t2)+y0 |
| A1 | 94.13 % |
| t1 | 0.12 ± 0.002 secs |
| A2 | 5.87 % |
| **t2** | **1.004 ± 0.037 secs** |

| Model | ExpDec2 |
|---|---|
| Equation | y=A1*(exp(-x/t1)+A2*(exp(-x/t2)+y0 |
| A1 | 94.1 % |
| t1 | 0.059 ± 0.0015 secs |
| A2 | 5.9 % |
| **t2** | **0.51 ± 0.0083 secs** |

# Figure 20 continued

| Model | ExpDec2 |
|-------|---------|
| Equation | y=A1*(exp(-x/t1)+A2*(exp(-x/t2)+y0 |
| A1 | 88.0 % |
| t1 | 0.58 ± 0.04 secs |
| A2 | 12.0 % |
| **t2** | **12.2 ± 0.75 secs** |

| Model | ExpDec2 |
|-------|---------|
| Equation | y=A1*(exp(-x/t1)+A2*(exp(-x/t2)+y0 |
| A1 | 99.8 % |
| t1 | 0.083 ± 72.9 secs |
| A2 | 0.2 % |
| **t2** | **14.75 ± 0.77 secs** |

## Figure 21

GS7-degen$^{P30,P32}$-$_T$AT647N,P28,P34

| Model | ExpDec2 |
|---|---|
| Equation | y=A1*(exp(-x/t1)+A2*(exp(-x/t2)+y0 |
| A1 | 95.5 % |
| t1 | 0.057 ± 0.00020 secs |
| A2 | 4.5 % |
| **t2** | **0.45 ± 0.0059 secs** |

GS7-mis4T-degen$^{P30,P32}$-$_T$AT647N,P28,P34

| Model | ExpDec2 |
|---|---|
| Equation | y=A1*(exp(-x/t1)+A2*(exp(-x/t2)+y0 |
| A1 | 97.1 % |
| t1 | 0.039 ± 0.00027 secs |
| A2 | 2.9 % |
| **t2** | **0.23 ± 0.0031 secs** |

| Name of Strand | DNA sequence |
|---|---|
| GSeq-CAP-(1-49)-B<sup>Bio,P49</sup> | 5' bio-GTG CCT AAA ATG TGA TCT AGA TCA CAT TTA TTG CGT AGA GCT CAC TGC C 3' (SEQ ID NO: 19) |
| GSeq-CAP-(1-49)-T<sup>Cy3B,P13</sup> | 5' GGC AGT GAG CTC TAC GCA ATA AAT GTG ATC TAG ATC ACA TTT TAG GCA C 3' (SEQ ID NO: 20) |
| GSeq-CAP-G<sup>P25</sup>-(1-49)-B<sup>Bio,P49</sup> | 5' bio-GTG CCT AAA ATG TGA TCT AGA TCA GAT TTA TTG CGT AGA GCT CAC TGC C 3' (SEQ ID NO: 21) |
| GSeq-CAP-C<sup>P25</sup>-(1-49)-T<sup>Cy3B,P13</sup> | 5' GGC AGT GAG CTC TAC GCA ATA AAT CTG ATC TAG ATC ACA TTT TAG GCA C 3' (SEQ ID NO: 22) |
| GSeq-CAP-(1-49)-B<sup>Bio,P49</sup> | 5' bio- GTG CCT AAA ATG TGA TCT AGA TCA CAT TTA TTG CGT AGA GCT CAC TGC C 3' (SEQ ID NO: 19) |
| GSeq-CAP-(1-20)-T<sup>Cy3B,P11</sup> | 5' GGC AGT GAG CTC TAC GCA AT 3' (SEQ ID NO: 23) |
| GSeq-CAP-(30-49)-T | 5' CTA GAT CAC ATT TTA GGC AC 3' (SEQ ID NO: 24) |
| GSeq-CAP-G<sup>P25</sup>-(1-49)-B<sup>Bio,P49</sup> | 5' bio- GTG CCT AAA ATG TGA TCT AGA TCA GAT TTA TTG CGT AGA GCT CAC TGC C 3' (SEQ ID NO: 21) |
| GSeq-CAP-(1-20)-T<sup>Cy3B,P11</sup> | 5' GGC AGT GAG CTC TAC GCA AT 3' (SEQ ID NO: 23) |
| GSeq-CAP-(30-49)-T | 5' CTA GAT CAC ATT TTA GGC AC 3' (SEQ ID NO: 24) |
| GS9-CAP-T<sup>AT647N,P21,P29</sup> | 5' AAA TGT GAT 3' |
| GS9-CAP-mis5C-T<sup>AT647N,P21,P29</sup> | 5' AAA TCT GAT 3' |

Figure 22

EP 3 682 033 B1

EP 3 682 033 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6440671 B1
- WO 0004193 A1
- WO 9719193 A2

### Non-patent literature cited in the description

- **TATON et al.** Scanometric DNA Array Detection with Nanoparticle Probes. *Science*, 2000, vol. 289 (5485), 1757-1760
- **VVEDENSKAYA et al.** *Molecular Cell*, 2017, vol. 60, 953-965
- **BERGER et al.** *Nucleic Acids Res.*, 01 August 2000, vol. 28 (15), 2911-2914
- **MOHSEN** ; **COLL**. *Acc Chem Res*, 2016
- **ROBERTS et al.** *Current Opinion in Chem Bio*, 1999
- **WATSON, J.** ; **CRICK, F.** A Structure for Deoxyribose Nucleic Acid. *Nature*, 1953, vol. 171 (4356), 737
- **SANGER, F.** ; **NICKLEN, S.** DNA sequencing with chain-terminating. *Proceedings of the National Academy of Sciences of the United States of America*, 1977, vol. 74 (12), 5463
- **MAXAM, A. M.** ; **GILBERT, W.** A new method for sequencing DNA. *Proceedings of the National Academy of Sciences of the United States of America*, 1977, vol. 74 (2), 560
- **EID, J.** ; **FEHR, A.** ; **GRAY, J** ; **LUONG, K** ; **LYLE, J** ; **OTTO, G.** ; **PELUSO, P.** ; **RANK, D**. Real-Time DNA Sequencing from Single Polymerase Molecules. *Science*, January 2009, vol. 323 (133), 139
- **CLARKE, J.** ; **WU, H.-C** ; **JAYASINGHE, L.** ; **PATEL, A.** ; **REID, S.** ; **BAYLEY, H**. Continuous base identification for single-molecule nanopore DNA sequencing. *Nature Nanotechnology*, April 2009, vol. 265, 270
- **RHOADS, A.** ; **AU, K. F**. PacBio Sequencing and Its Applications.. *Genomics Proteomics Bioinformatics*, 2015, vol. 13 (278), 289
- *Oxford Nanopore Technologies*, 23 August 2017
- **DRMANAC, R** ; **BRUKNER, I.** ; **LABAT, I.** ; **BRUKNER, I.** ; **CRKVENJAKOV, R**. Sequencing of megabase plus DNA by hybridization : Theory of the Method. *Genomics*, 1989, vol. 7543 (114), 128

- **BAINS, W.** ; **SMITH, G**. A Novel Method for Nucleic Acid Sequence Determination. *Journal of Theoretical Biology*, 1988, vol. 135 (303), 307
- **LYSOV, V.** ; **FLORETIEV, V** ; **KHORLYN, A.** ; **KHRAPKO, K** ; **SHICK, V.** ; **MIRZABEKOV, A**. DNAsequencing by hybridisation with oligonucleotides. *Dokl. Acad. Sci. USSR*, 1988, vol. 303 (1508), 1511
- **JUNGMANN, R.** ; **STEINHAUER, C** ; **SCHEIBLE, M** ; **KUZYK, A** ; **TINNEFELD, P.** ; **SIMMEL, F. C**. Single-molecule kinetics and super-resolution microscopy by fluorescence imaging of transient binding on DNA origami.. *Nano Letters*, 2010, vol. 10 (11), 4756
- **HEILEMANN, M.** ; **HWANG, L. C.** ; **LYMPEROPOULOS, K.** ; **KAPANIDIS, A. N.** Single-Molecule FRET Analysis of Protein DNA Complexes. *In Methods in Molecular Biology*, 2009, vol. 543 (503), 521
- **JOO, C.** ; **HA, T**. Single-Molecule FRET with Total Internal Reflection Microscopy. *In Single-Molecule Techniques: A Laboratory Manual*, 2008, vol. 3, 36
- **KAPANIDIS, A. N.** ; **LEE, N. K** ; **LAURENCE, T. A.** ; **DOOSE, S** ; **MARGEAT, E.** ; **WEISS, S**. Fluorescence-aided molecule sorting: analysis of structure and interactions by alternating-laser excitation of single molecules. *Proceedings of the National Academy of Sciences of the United States of America*, 2004, vol. 101 (24), 8936
- **KAPANIDIS, A.** ; **JING, B.** ; **CRAWFORD, R**. *Compact Microscope*, 2015
- **DUPUIS, N. F.** ; **HOLMSTROM, E. D.** ; **NESBITT, D. J.** Single-molecule kinetics reveal cation-promoted DNA duplex formation through ordering of single-stranded helices. *Biophysical Journal*, 2013, vol. 105 (3), 756
- **CISSE, I. I.** ; **KIM, H.** ; **HA, T**. A rule of seven in Watson-Crick base-pairing of mismatched sequences. *Nature Structural & Molecular Biology*, 2012, vol. 19 (6), 623
- **LAKOWICZ, J**. Principles of Fluorescence Spectroscopy. Springer, 2006

- **MELCHIOR, W. B.** ; **HIPPEL, P**. Alteration of the Relative Stability of dA-dT and dG-dC Base Pairs in DNA. *Proceedings of the National Academy of Sciences of the United States of America*, 1973, vol. 70 (2), 298
- **EBRIGHT, R. H.** ; **EBRIGHT, Y. W** ; **GUNASEKERA, A.** ; **BRUNSWICK, N.** Consensus DNA site for the Escherichia coli catabolite gene activator protein (CAP): CAP exhibits a 450-fold higher a_nity for the consensus DNA site than for the E.coli lac DNA site. *Nucleic Acids Research*, 1989, vol. 17 (23), 16
- **LAWSON, C. L.** ; **SWIGON, D** ; **MURAKAMI, K. S** ; **DARST, S. A** ; **HELEN, M.** ; **EBRIGHT, R. H.** Catabolite activator protein (CAP): DNA binding and transcription activation. *Current Opinion in Structural Biology*, 2004, vol. 14 (1), 10
- **CRAWFORD, R** ; **KELLY, D. J.** ; **KAPANIDIS, A. N**. A Protein Biosensor That Relies on Bending of Single DNA Molecules. *Chemphyschem*, 2012, vol. 13 (918), 922
- **BAUER, D. L. V.** ; **PLOCHOWIETZ, A.** ; **DULIN, D** ; **MALINEN, A. M** ; **BAKERMANS, J. J. W.** ; **KALLER, M** ; **KAPANIDIS, A. N.** Sequence-Encoded Pause Signals Modulate Initial Transcription in Bacteria. *Manuscript in Preparation*, 2017